(19) Europäisches Patentamt
European Patent Office
Office européen des brevets

(11) Numéro de publication: **0 429 370 B1**

# (12) FASCICULE DE BREVET EUROPEEN

(45) Date de publication de fascicule du brevet: **04.01.95**

(51) Int. Cl.⁶: **C07D 333/20**, C07D 333/24, A61K 31/38

(21) Numéro de dépôt: **90403310.7**

(22) Date de dépôt: **23.11.90**

(54) **Nouveaux dérivés du thiophène, leur procédé de préparation et les compositions pharmaceutiques les renfermant.**

(30) Priorité: **24.11.89 FR 8915458**

(43) Date de publication de la demande:
**29.05.91 Bulletin 91/22**

(45) Mention de la délivrance du brevet:
**04.01.95 Bulletin 95/01**

(84) Etats contractants désignés:
**AT BE CH DE DK ES FR GB GR IT LI LU NL SE**

(56) Documents cités:
**EP-A- 0 155 524**

**JOURNAL OF MEDICINAL CHEMISTRY, vol. 27, no. 3, mars 1984, pages 390-397,American Chemical Society; M.M. GOODMAN et al.: "Synthesis and evaluation ofradioiodinated terminal p-iodophenyl-substituted alpha- and beta-methyl-branched fatty acids"**

(73) Titulaire: **ADIR ET COMPAGNIE**
**1 rue Carle Hébert**
**F-92415 Courbevoie Cédex (FR)**

(72) Inventeur: **Wierzbicki, Michel**
**8 Rés. du Chemin Pavé**
**F-78620 L'Etang La Ville (FR)**
Inventeur: **Sauveur Frédéric**
**16 avenue Foch**
**F-95100 Argenteuil (FR)**
Inventeur: **Bonnet, Jacqueline**
**19 rue Charcot**
**F-75013 Paris (FR)**
Inventeur: **Brisset, Martine**
**6 Place de l'Ancienne Boucherie**
**F-14000 Caen (FR)**
Inventeur: **Tordjman, Charles**
**21 Ouai Le Gallo**
**F-92100 Boulogne (FR)**

(74) Mandataire: **Reverbori, Marcelle**
**ADIR**
**1, rue Carle Hébert**
**F-92415 Courbevoie Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention a pour objet les nouveaux dérivés du thiophène, leur procédé de préparation et les compositions pharmaceutiques les renfermant.

Elle concerne particulièrement les dérivés du thiophène de formule générale I :

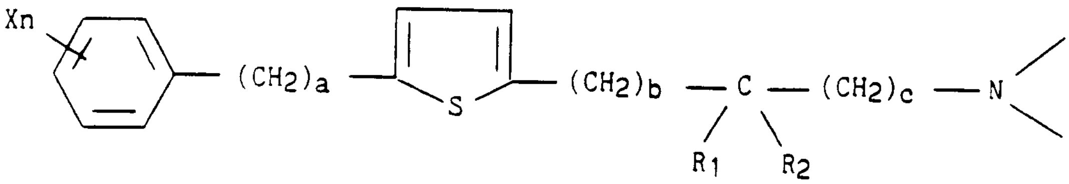

(I)

dans laquelle :

- X représente un atome d'hydrogène ou d'halogène, un radical alkyle ou alkoxy ayant chacun de 1 à 5 atomes de carbone en chaîne droite ou ramifiée ou un radical dialkylamino dans lequel chaque groupe alkyle renferme de 1 à 5 atomes de carbone ;
- n représente 1 ou 2 ;
- a représente un nombre entier de 2 à 6 inclus ;
- b représente 2 ou 3 ;
- c représente 1 ou 2, de telle sorte que b + c = 4 ;
  $R_1$ et $R_2$, identiques ou différents, représentent chacun un atome d'hydrogène ou un radical alkyle ayant de 1 à 5 atomes de carbone, ou
- $R_1$ et $R_2$ forment ensemble avec l'atome de carbone auquel ils sont liés un cycle hydrocarboné renfermant de 3 à 6 atomes de carbone ; et
- R et R', identiques ou différents, représentent chacun un atome d'hydrogène ou un radical alkyle ayant de 1 à 5 atomes de carbone, ou
- R et R' forment ensemble avec l'atome d'azote auquel ils sont liés un radical hétérocyclique penta ou hexagonal renfermant éventuellement un atome d'oxygène ou un deuxième atome d'azote lequel peut être lui-même substitué par un radical alkyle renfermant de 1 à 5 atomes de carbone en chaîne droite ou ramifiée, ou par un radical arylalkyle dans lequel le groupe alkyle renferme de 1 à 5 atomes de carbone et le groupe aryle est éventuellement mono- ou poly- substitué par un atome d'halogène ou un radical alkyle ou alkyloxy ayant chacun de 1 à 5 atomes de carbone.

L'état antérieur de la technique le plus proche de la présente invention est illustré notamment par :

le brevet DE.3.407.510 qui concerne, entre autres, l'acide de formule :

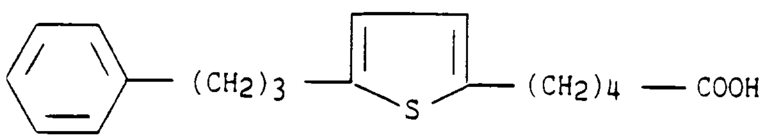

lequel a une activité anti-inflammatoire ;

F.F.KNAPP et coll. Journal of nuclear Medicine, 27 (4), 521-31 (1986) qui décrit notamment les esters de formule :

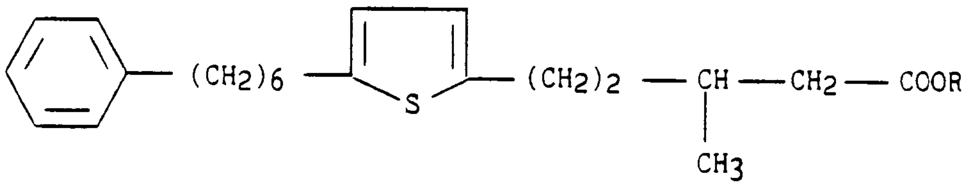

et M.M. GOODMANN et coll. J. Med. Chem. 27, 390-97 (1984) qui décrit, entre autres, le dérivé de formule :

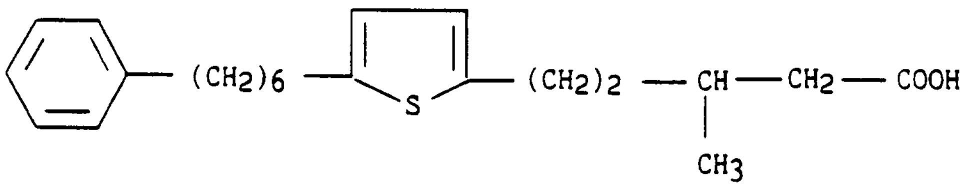

Aucune de ces références ne décrit ni suggère les amines de formule générale I objet de la présente invention, lesquelles amines possèdent une activité anti résorbante osseuse, nullement mentionnée pour les acides et esters structurellement les plus proches de l'état antérieur de la technique.

La présente invention a également pour objet le procédé de préparation des dérivés de formule générale I caractérisé en ce que :

l'on transforme, au moyen par exemple de chlorure de thionyle, l'acide de formule générale II :

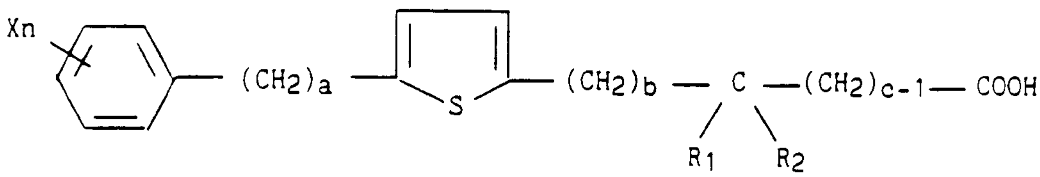

(II)

dans laquelle X, n, a, b, c, $R_1$ et $R_2$ ont les significations précédemment définies,
en chlorure d'acide correspondant de formule générale III :

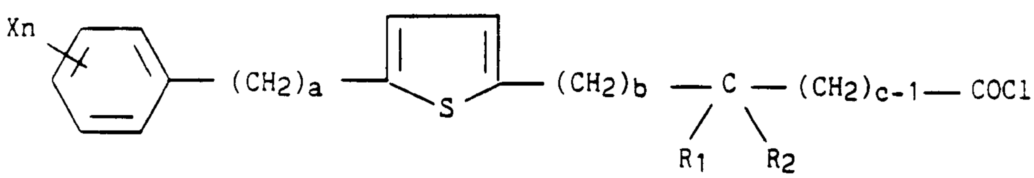

(III)

dans laquelle X, n, a, b, c, $R_1$ et $R_2$ ont les significations précédemment définies,
lequel chlorure d'acide sert à acyler l'amine de formule générale IV :

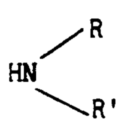

(IV)

dans laquelle R et R' ont les significations précédemment définies,
et l'on réduit l'amide ainsi obtenue de formule générale V :

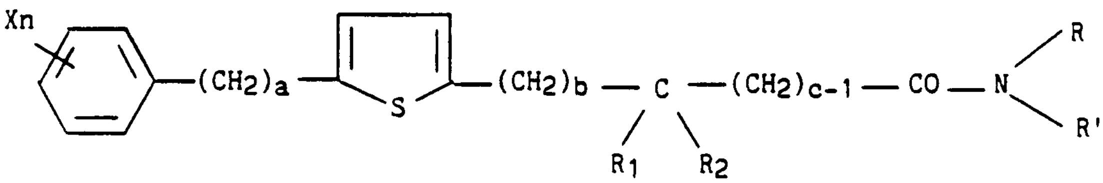

(V)

dans laquelle X, n, a, b, c, $R_1$, $R_2$, R et R' ont les significations précédemment définies.

Il est particulièrement adéquat d'effectuer cette réduction au moyen de $Li\ Al\ H_4$ en opérant à reflux dans un solvant adéquat tel que, par exemple, l'éther.

L'acide de formule générale II de départ a été préparé soit à partir de l'acide de formule générale :

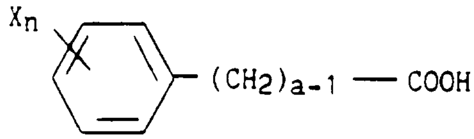

transformé en chlorure d'acide correspondant de formule générale :

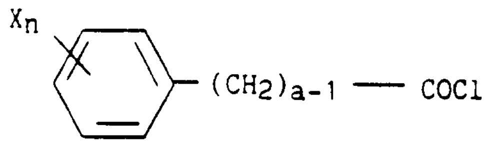

lequel, avec une quantité équimoléculaire de thiophène est soumis à la réaction de Friedel-Crafts en présence de $Al\ Cl_3$ ou $Sn\ Cl_4$ pour donner le composé de formule générale A :

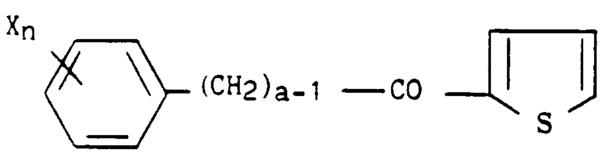

(A)

lequel soumis à la réduction de Wolff-Kishner en présence de potasse et d'hydrazine, donne le composé de formule générale B :

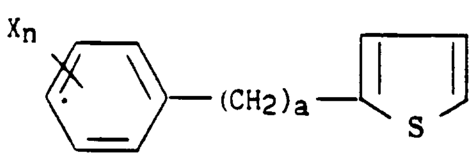

(B)

qui soumis à la réaction de Friedel-Crafts avec l'anhydride glutarique substitué de formule générale :

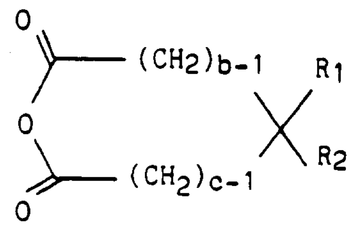

donne le composé de formule générale C :

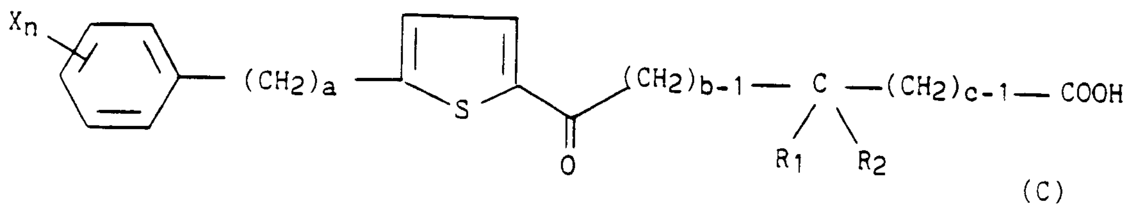

(C)

qui par réduction de Wolff-Kishner donne l'acide de formule générale II ;
soit à partir de thiophène et de l'anhydride glutarique substitué de formule générale :

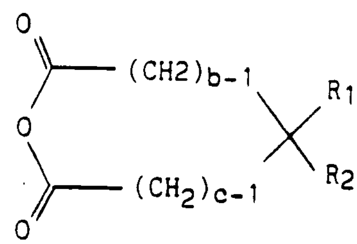

qui soumis à la reaction de Friedel-Crafts en présence d'Al $Cl_3$ dans le nitrobenzène donnent le composé de formule générale D :

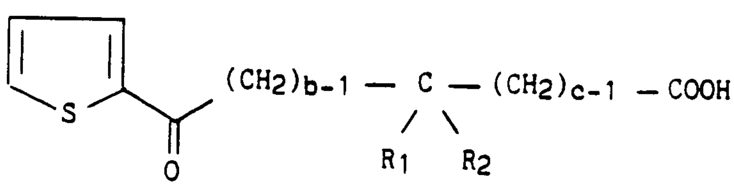

(D)

lequel réduit selon la méthode de Wolff-Kishner, au moyen d'hydrazine et de potasse, donne l'acide de formule générale E :

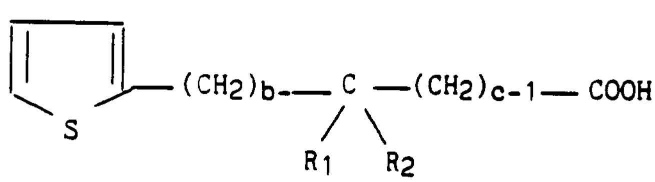

(E)

qui estérifié par $CH_3$ OH/APTS donne l'ester de formule générale F :

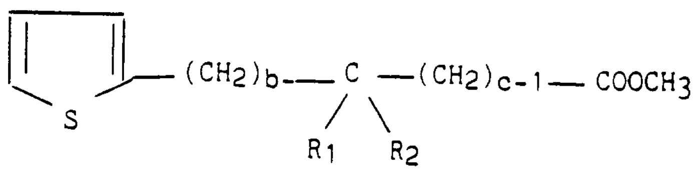

(F)

lequel soumis à l'acylation de Friedel-Crafts, avec Sn $Cl_4$ et $CH_2$ $Cl_2$ donne l'ester de formule générale G :

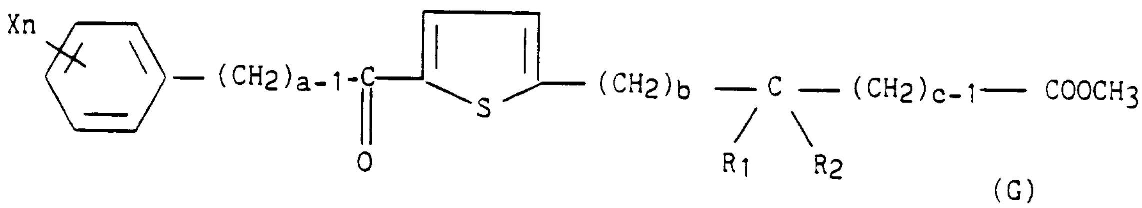

(G)

qui par réduction de Wolff-Kishner, au moyen d'hydrazine et de potasse donne l'acide de formule générale II ;

étant admis que, dans chacune des formules ci-dessus les variables X, n, a, b, c, $R_1$, $R_2$ R et R' ont les significations précédemment définies.

Les dérivés de formule générale I donnent des sels avec les acides physiologiquement tolérables - sels qui sont, à ce titre, inclus dans la présente invention.

Les amides de formule générale V, produits intermédiaires dans la synthèse des dérivés de formule générale I, sont des produits nouveaux qui sont, à ce titre, inclus dans la présente invention.

Les dérivés de la présente invention possèdent des propriétés pharmacologiques et thérapeutiques intéressantes notamment sur le métabolisme osseux.

Sur un test d'hyperrésorption osseuse, à l'acide rétinoïque, pratiqué sur calvaria de souris en culture selon une technique inspirée de la méthode de J.J. REYNOLDS et Coll. - Calc. Tiss. Res. 4, 339-49, (1970)- les dérivés de la présente invention ont montré une activité antirésorbante allant de 5 à 20%, pour des concentrations molaires comprises entre $10^{-6}$ et $5.10^{-5}$.

Par exemple, le composé objet de l'exemple 8 a présenté sur ce test l'activité traduite par la courbe suivante :

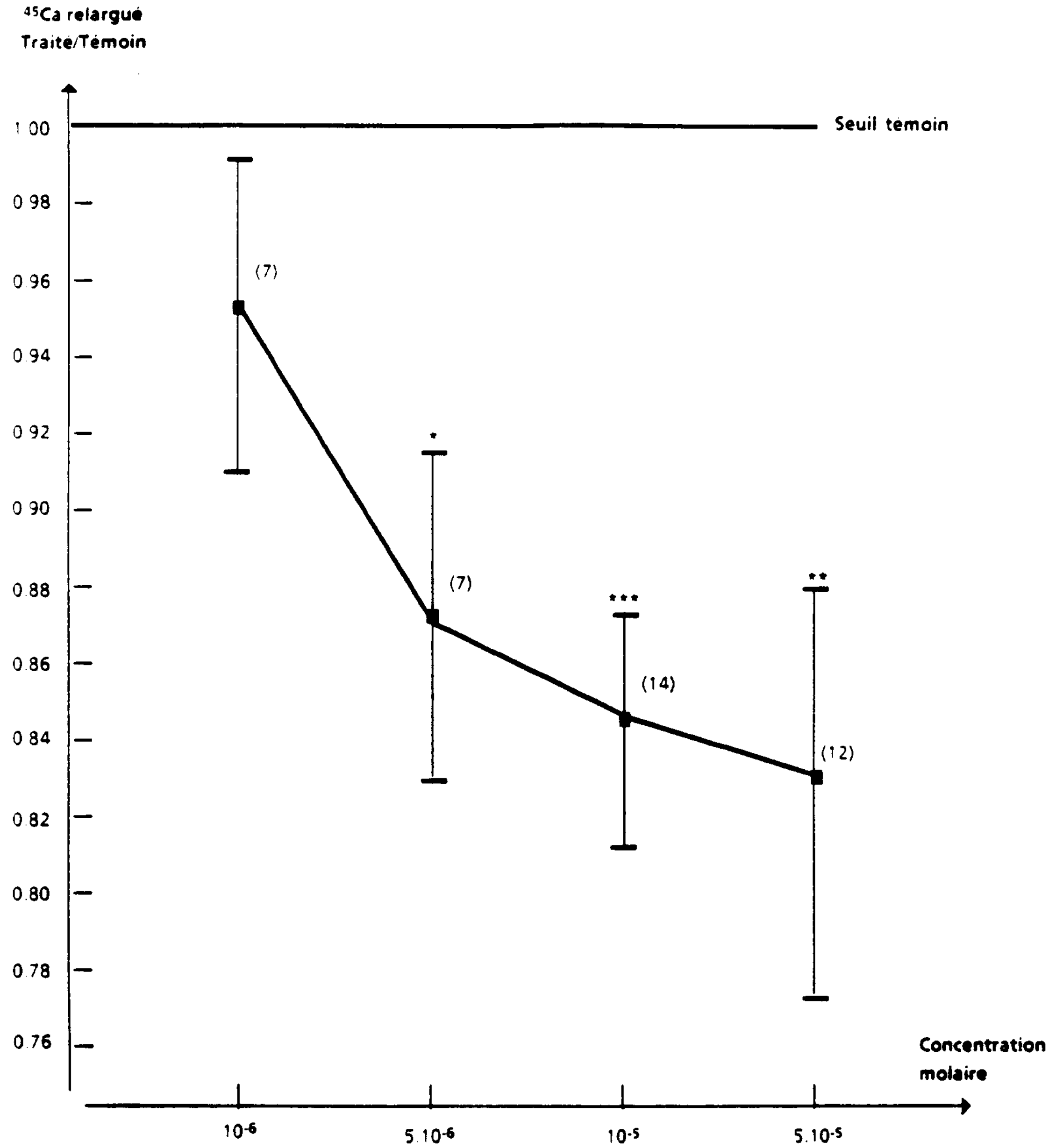


**Chaque valeur en ordonnée est la valeur moyenne ± esm (le nombre de calvaria traitées étant indiqué entre parenthèses). Comparaison à la valeur moyen témoin *P <0.05, **P<0.01, ***P<0.001.**

Par ailleurs, certains composés de la présente invention et notamment le composé objet de l'exemple 9 ont exercé une activité stimulante sur la formation osseuse in vitro (incorporation de proline-$H^3$ dans le tissu osseux de calvaria de souris, mis en culture selon la technique décrite par M.C. MEIKLE et coll. -Calcif.Tissue Int 34, 359-64, (1982) - pour des concentrations de $10^{-6}$ à $10^{-5}$M.

Ces composés ont montré ces intéressantes propriétés sur le métabolisme osseux, tout en conservant une activité inhibitrice de libération des métabolites lipoxygénase-dépendants de l'acide arachidonique avec des $IC_{50}$ comprises entre $10^{-6}$ et $10^{-5}$M. (technique d'étude de la biosynthèse des métabolites de l'acide arachidonique par polynucléaires de rat stimulés à l'ionophore A 23187 ; mesure par HPLC avec détection radioactive).

D'autre part ces composés ne sont pas toxiques après administration aigüe par voie orale chez la souris ($DL_{50}$≧, 1000mg/kg).

En conséquence, les propriétés pharmacologiques et l'absence de toxicité des composés de la présente invention permettent leur utilisation dans les pathologies caractérisées par une perte du tissu osseux, comme l'ostéoporose, la maladie de Paget, la parodontite et la polyarthrite rhumatoïde.

La présente invention a également pour objet les compositions pharmaceutiques contenant comme principe actif un composé de formule générale I ou un de ses sels physiologiquement tolérable, mélangé ou associé à un excipient pharmaceutique approprié, comme par exemple, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent revêtir, par exemple, la forme de comprimés, dragées, gélules, suppositoires, solutions injectables ou buvables et être selon les cas administrées par voie orale, rectale ou parentérale.

Les exemples suivants ilustrent la présente invention.

**I) Synthèse des matières premières de formule générale II :**

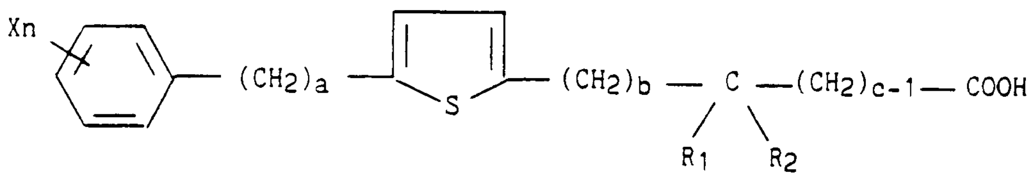

(II)

**A) Première méthode :**

a) Préparation des dérivés de formule générale A :

150,18g (1 mole) d'acide hydrocinnamique sont ajoutés par fractions à 95ml (1,3 mole) de chlorure de thionyle à froid sous agitation. Après une heure d'agitation le milieu réactionnel est porté à 40 ° C jusqu'à fin du dégagement gazeux. L'excès de chlorure de thionyle est distillé sous pression réduite.

Le chlorure d'acide ainsi obtenu et 82,55g (1,1 mole) de thiophène sont mis en solution dans 1,21 de dichlorométhane anhydre. La température est amenée à 0 ° C sur saumure. 312,6g (1,2 mole) de chlorure d'étain sont coulés goutte à goutte sous vive agitation en maintenant la température à 0 ° C. Après une heure à 0 ° C, le milieu réactionnel est agité pendant 12 heures à température ambiante, hydrolysé sur 600ml d'HCl en solution 6 N, décanté et la phase aqueuse est extraite par 4 fois 100ml de dichlorométhane. Les phases organiques sont filtrées sur célite, lavées par une solution N d'HCl, une solution N de Na OH, et par l'eau, puis séchées sur sulfate de magnésium et décolorées au noir animal. Le solvant distillé laisse 216g d'huile brute.

Ce produit brut peut être distillé pour donner le (phényl-3 propanoyl)-2 thiophène, Eb/$10^{-2}$ torr = 135 ° C

Ont ainsi été préparés les produits regroupés dans le tableau 1 ci-après.

**Tableau 1 : Dérivés de formule :**

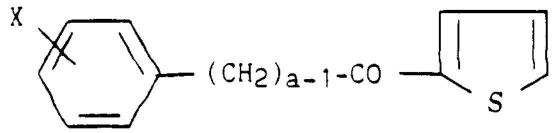

| a | X |
|---|---|
| 2 | H |
| 2 | p.$CH_3$ |
| 3 | H |
| 3 | m.F |
| 3 | p. Cl |
| 3 | m. $CH_3$ |
| 3 | p.$CH_3$ |
| 3 | p. $OCH_3$ |
| 4 | H |
| 4 | p. $CH_3$ |
| 6 | H |

b) Préparation des dérivés de formule générale B :

90g (0,416 mole) de (phényl-3 propanoyl)-2 thiophène sont placés dans 500ml de triéthylène glycol. La dissolution est obtenue par chauffage à 70 °C sous agitation. On ajoute en une fois 62g (1,56 mole) d'hydrate d'hydrazine (80%) et on porte la température à 100 °C. Puis 75g (1,335 mole) de potasse sont ajoutés rapidement et le reflux est maintenu pendant 45mn.

L'eau formée est distillée jusqu'à ce que la température atteigne 210-220 °C. Le reflux est ainsi maintenu, pendant 2 heures puis la température du milieu réactionnel est amenée à 40 °C. Le mélange est hydrolysé par 400ml d'eau et 100ml d'HCl concentré, puis extrait successivement par 250ml puis 3 fois 80ml d'éther. La phase organique est lavée successivement par une solution normale d'HCl, de l'eau, une solution aqueuse saturée de bicarbonate, puis de l'eau et enfin séchée sur Mg $SO_4$ et décolorée au noir animal. Le solvant distillé laisse 70g d'huile brute de (phényl-3 propyl)-2 thiophène.

Ont ainsi été préparés les produits regroupés dans le tableau 2 ci-après.

**Tableau 2 : Dérivés de formule :**

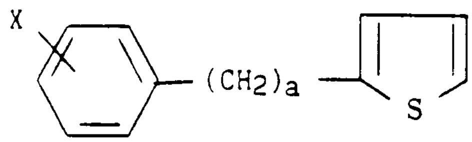

| a | X |
|---|---|
| 2 | H |
| 2 | p.$CH_3$ |
| 3 | H |
| 3 | m.F |
| 3 | p. Cl |
| 3 | m. $CH_3$ |
| 3 | p.$CH_3$ |
| 3 | p. $OCH_3$ |
| 3 | p.OH |
| 4 | H |
| 4 | p.CH3 |
| 6 | H |

c) Préparation des dérivés de formule générale C :

35g (0,173 mole) de (phényl-3 propyl)-2 thiophène et 27,05g (0,190 mole) d'anhydride diméthyl-3,3 glutarique sont mis en solution dans 500ml de nitrobenzène. La température du milieu réactionnel est amenée à 0 ° C et 57,67g (0,433 mole) de chlorure d'aluminium sont ajoutés par fractions, sous vive agitation en maintenant la température à 5 ° C.

Après 1 heure à 5 ° C, l'agitation est maintenue 12 heures à température ambiante.

Le mélange est versé sur 500g de glace additionnée de 50ml d'HCl concentré. L'hydrolyse est poursuivie pendant 2 heures sous agitation, puis la phase organique est grossièrement décantée et le nitrobenzène entraîné à la vapeur.

L'acide est extrait par 3 rois 100ml d'éther, la phase organique est séchée sur Mg $SO_4$ et le solvant distillé. Le résidu est dissous dans 200ml de bicarbonate de sodium saturé, extrait par 50ml d'éther et décoloré au noir animal. La solution aqueuse est acidifiée par HCl aqueux 4N et extraite par 3 fois 100ml d'éther.La phase organique lavée par HCl aqueux 1N, puis de l'eau, est séchée 3 heures sur Mg $SO_4$. Le solvant distillé laisse 55g d'huile jaune pâle qui cristallise lentement en (phényl-3 propyl)-5 (diméthyl-3,3 glutaryl)-2 thiophène. Ont ainsi été préparés les produits regroupés dans le tableau 3 ci-après.

**Tableau 3** : Dérivés de formule :

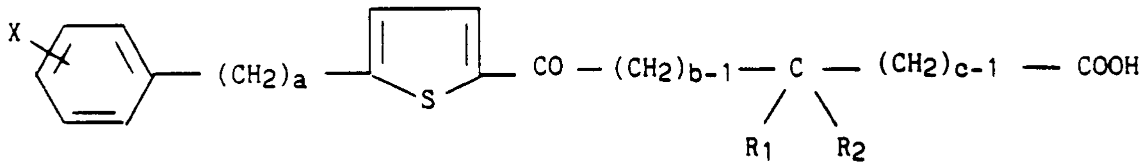

| X | a | b | c | $R_1$ | $R_2$ |
|---|---|---|---|---|---|
| H | 2 | 2 | 2 | $CH_3$ | $CH_3$ |
| p.$CH_3$ | 2 | 2 | 2 | $CH_3$ | $CH_3$ |
| H | 3 | 2 | 2 | H | H |
| p.$CH_3$ | 3 | 2 | 2 | H | H |
| H | 3 | 2 | 2 | $CH_3$ | H |
| p.$CH_3$ | 3 | 2 | 2 | $CH_3$ | H |
| H | 3 | 2 | 2 | $CH_3$ | $CH_3$ |
| p.$CH_3$ | 3 | 2 | 2 | $CH_3$ | $CH_3$ |
| m.$CH_3$ | 3 | 2 | 2 | $CH_3$ | $CH_3$ |
| H | 3 | 2 | 2 | 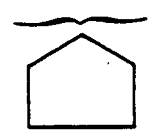 | |
| H | 3 | 3 | 1 | $CH_3$ | $CH_3$ |
| p.$CH_3$ | 3 | 3 | 1 | $CH_3$ | $CH_3$ |
| H | 4 | 2 | 2 | $CH_3$ | $CH_3$ |
| p.$CH_3$ | 4 | 2 | 2 | $CH_3$ | $CH_3$ |
| H | 6 | 2 | 2 | $CH_3$ | $CH_3$ |

d) Préparation des dérivés de formule générale II :

50g (0,145 mole) de (phényl-3 propyl)-5 (diméthyl-3,3 glutaryl)-2 thiophène sont mis en solution dans 250ml de triéthylène glycol.

La température est portée à 70 ° c et on ajoute 28,5mg (0,536 mole) d'hydrate d'hydrazine (80%).

La température est amenée à 100 ° C et on ajoute 24,4g (0,435 mole) de potasse. Le reflux est maintenu 45 minutes, puis l'eau est distillée jusqu'à ce que la température atteigne 210-220 ° C.

Le reflux est alors maintenu 2 heures.

Le milieu réactionnel est hydrolysé par 300ml d'eau additionnée de 35ml d'HCl concentré, puis extrait par 3 fois 100ml d'éther.

La phase organique est lavée par HCl aqueux N puis de l'eau, séchée sur Mg $SO_4$ et décolorée au noir animal.

Le solvant distillé laisse 41,5g d'huile incolore qui cristallise lentement en (phényl-3 propyl)-5 (carboxy-4 diméthyl-3,3 butyl)-2 thiophène.

Ont ainsi été préparés les produits regroupés dans le tableau 4 ci-après :

**Tableau 4 : Dérivés de formule :**

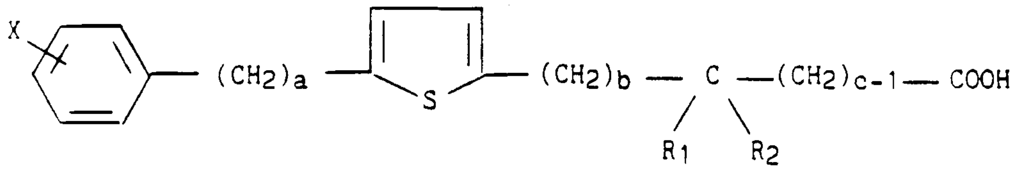

| X | a | b | c | $R_1$ | $R_2$ |
|---|---|---|---|---|---|
| H | 2 | 2 | 2 | $CH_3$ | $CH_3$ |
| p.$CH_3$ | 2 | 2 | 2 | $CH_3$ | $CH_3$ |
| H | 3 | 2 | 2 | H | H |
| p.$CH_3$ | 3 | 2 | 2 | H | H |
| H | 3 | 2 | 2 | $CH_3$ | H |
| p.$CH_3$ | 3 | 2 | 2 | $CH_3$ | H |
| H | 3 | 2 | 2 | $CH_3$ | $CH_3$ |
| p.$CH_3$ | 3 | 2 | 2 | $CH_3$ | $CH_3$ |
| m.$CH_3$ | 3 | 2 | 2 | $CH_3$ | $CH_3$ |
| H | 3 | 2 | 2 | 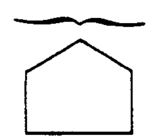 | |
| H | 3 | 3 | 1 | $CH_3$ | $CH_3$ |
| p.$CH_3$ | 3 | 3 | 1 | $CH_3$ | $CH_3$ |
| H | 4 | 2 | 2 | $CH_3$ | $CH_3$ |
| p.$CH_3$ | 4 | 2 | 2 | $CH_3$ | $CH_3$ |
| H | 6 | 2 | 2 | $CH_3$ | $CH_3$ |

**B) Deuxième méthode :**

a) préparation des dérivés de formule générale D :

42,07g(0,5 mole) de thiophène et 71,07g (0,55 mole) d'anhydride diméthyl-3,3 glutarique sont mis en solution dans 1500ml de nitrobenzène. Le milieu réactionnel est refroidi à 0-2 °C sur bain de saumure et 166,67g (1,25 mole) de chlorure d'aluminium sont ajoutés par fractions sous vive agitation en maintenant la température du milieu réactionnel à une valeur inférieure à 5 °C. La réaction est poursuivie 30 minutes à une température de 0-5 °C puis complétée à température ambiante pendant 10 heures.

Le milieu réactionnel est hydrolysé sur 31 ml d'un mélange eau/glace. Après addition de 217ml d'HCl concentré, la phase organique est décantée, le nitrobenzène entraîné à la vapeur et la phase aqueuse résiduelle extraite par 3 fois 200ml d'éther éthylique. Les phases éthérées réunies sont lavées par une

solution aqueuse d'HCl normale puis par de l'eau. Après traitement sur sulfate de magnésium et noir animal, les solvants distillés laissent 106,5g d'une huile jaune pâle qui cristallise lentement (Rendement : 94%).

Le produit peut être recristallisé dans l'eau pour donner après filtration et séchage le (diméthyl-3,3 glutaryl)-2 thiophène sous forme d'un solide blanc, P.F. : 70 °C.

Ont ainsi été préparés les produits regroupés dans le tableau 5 ci-après.

**Tableau 5 : Dérivés de formule :**

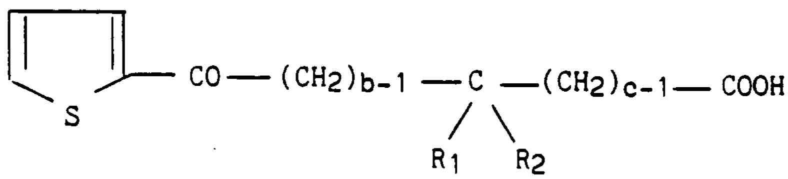

| b | c | $R_1$ | $R_2$ |
|---|---|---|---|
| 2 | 2 | $CH_3$ | $CH_3$ |
| 2 | 2 | $CH_3$ | H |
| 2 | 2 | H | H |
| 1 | 3 | $CH_3$ | $CH_3$ |
| 3 | 1 | $CH_3$ | $CH_3$ |

b) Préparation des dérivés de formule générale E :

17,5g (0,0773 mole) de (diméthyl-3,3 glutaryl)-2 thiophène sont mis en suspension dans 100ml de triéthylèneglycol. La température est portée à 70 °C et l'agitation est maintenue jusqu'à dissolution complète. 10,83g (0,27 mole) d'hydrate d'hydrazine à 80% sont ajoutés. La température est portée à 100 °C puis on ajoute 13g (0,23 mole) de potasse en pastilles.

On maintient le reflux 30 minutes puis on distille l'eau formée en une heure environ ; la température s'élève jusqu'à 200-210 °C, la réaction est complète après une heure à cette température.

Après avoir refroidi le milieu réactionnel à 20 °C, on hydrolyse par 300ml d'eau et on neutralise à l'acide chlorhydrique concentré.

La phase aqueuse est extraite par 3 fois 100ml d'éther éthylique, puis, les phases éthérées sont lavées par une solution aqueuse normale d'HCl, puis par de l'eau. Après traitement par le sulfate de magnésium anhydre et le noir animal, la distillation du solvant laisse 14,37g d'une huile jaune pâle qui cristallise lentement en acide (thiényl-2)-5 diméthyl-3,3 pentanoïque. Rendement : 80%.

Ont ainsi été préparés les produits regroupés dans le tableau 6 ci-après :

**Tableau 6 : Dérivés de formule :**

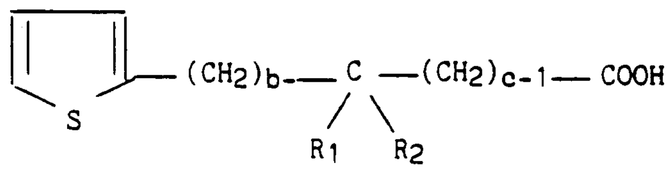

| b | c | $R_1$ | $R_2$ |
|---|---|---|---|
| 2 | 2 | $CH_3$ | $CH_3$ |
| 2 | 2 | $CH_3$ | H |
| 2 | 2 | H | H |
| 1 | 3 | $CH_3$ | $CH_3$ |
| 3 | 1 | $CH_3$ | $CH_3$ |

c) Préparation des dérivés de formule générale F :

13g (0,0612 mole) d'acide (thiényl-2)-5 diméthyl-3,3 pentanoïque sont mis en solution dans 200ml de méthanol anhydre en présence d'une quantité catalytique (0,025g) d'acide p.toluènesulfonique.

Le milieu est porté puis maintenu au reflux pendant 20 heures. Après vérification de l'absence d'acide de départ (par chromatographie en couche mince), on distille le méthanol et reprend le résidu dans l'éther éthylique. Après lavage de la phase éthérée à l'eau, traitement sur Mg $SO_4$ anhydre puis noir animal, la distillation du solvant laisse une huile brute qui est chromatographiée ($SiO_2/CH_2Cl_2$) pour laisser 12,25g d'une huile incolore. Rendement : 88%.

Ont ainsi été préparés les esters méthyliques des acides du tableau 6, c'est à dire les produits regroupés dans le tableau 7, ci-après :

**Tableau 7 : Dérivés de formule :**

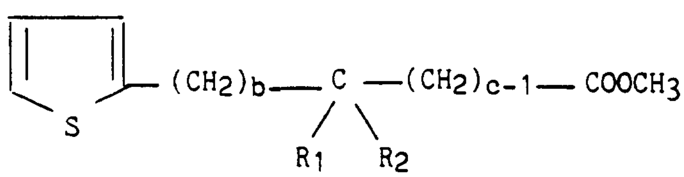

| b | c | $R_1$ | $R_2$ |
|---|---|---|---|
| 2 | 2 | $CH_3$ | $CH_3$ |
| 2 | 2 | $CH_3$ | H |
| 2 | 2 | H | H |
| 1 | 3 | $CH_3$ | $CH_3$ |
| 3 | 1 | $CH_3$ | $CH_3$ |

d) Préparation des dérivés de formule générale G :

7g (0,0309 mole) d'ester méthylique de l'acide (thiényl-2)-5 diméthyl-3,3 pentanoïque et un équivalent de chlorure de l'acide p.fluorophényl propanoïque - préparé à partir de 5,2g (0,0309 mole) d'acide p.fluorophénylpropanoïque et 3,36ml de $SOCl_2$ - sont mis en solution dans 175ml de chlorure de méthylène anhydre. A la solution maintenue à 0 ° C on ajoute goutte à goutte 4,52ml (0,0386 mole) de chlorure d'étain sous vive agitation. La réaction est complète après 30 mn à 0 ° C, puis une nuit à température ambiante.

Le milieu réactionnel est hydrolysé sur 500ml d'un mélange eau-glace acidifié par 50ml d'HCl concentré. Après 4 heures d'agitation, la phase organique est décantée et la phase aqueuse est extraite par 3 fois 100ml de dichlorométhane.

Les phases organiques, lavées par une solution aqueuse normale d'HCl, une solution aqueuse saturée de bicarbonate de sodium puis de l'eau, puis traitées par Mg $SO_4$ et le noir animal et concentrées à sec laissent 11,06g d'une huile brute qui, après chromatographie ($SiO_2/CH_2Cl_2$) donnent 10,48g d'une huile incolore. Rendement : 90,5%.

Ont ainsi été préparés les produits regroupés dans le tableau 8 ci-après :

**Tableau 8** : Dérivés de formule :

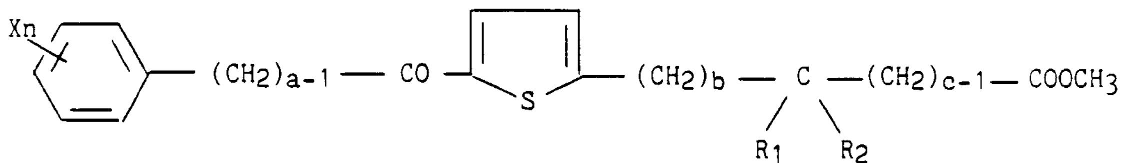

| X | a | b | c | R1 | R2 |
|---|---|---|---|---|---|
| H | 3 | 2 | 2 | $CH_3$ | $CH_3$ |
| p.F | 3 | 2 | 2 | $CH_3$ | $CH_3$ |
| p.F | 3 | 2 | 2 | $CH_3$ | H |
| m.F | 3 | 2 | 2 | $CH_3$ | $CH_3$ |
| o.Cl | 3 | 2 | 2 | $CH_3$ | $CH_3$ |
| H | 6 | 2 | 2 | $CH_3$ | $CH_3$ |
| 2,4 di$CH_3$ | 3 | 2 | 2 | $CH_3$ | H |
| 2,4 di$CH_3$ | 3 | 2 | 2 | $CH_3$ | $CH_3$ |
| m.F | 3 | 2 | 2 | $CH_3$ | H |
| p.$CH_3$ | 4 | 2 | 2 | $CH_3$ | H |
| p.$CH_3$ | 4 | 2 | 2 | $CH_3$ | $CH_3$ |
| 2,5 di$CH_3$ | 3 | 2 | 2 | $CH_3$ | $CH_3$ |
| 2,5 di$CH_3$ | 3 | 2 | 2 | $CH_3$ | H |
| p.$CH_3$ | 3 | 2 | 2 | H | H |
| p.$CH_3$ | 3 | 1 | 3 | $CH_3$ | $CH_3$ |
| p.$CH_3$ | 3 | 3 | 1 | $CH_3$ | $CH_3$ |
| p.-$CH(CH_3)_2$ | 3 | 2 | 2 | $CH_3$ | H |
| p.-$CH(CH_3)_2$ | 3 | 2 | 2 | $CH_3$ | $CH_3$ |
| p.-$(CH_2)_4$-$CH_3$ | 3 | 2 | 2 | $CH_3$ | H |
| p.-$(CH_2)_4$-$CH_3$ | 3 | 2 | 2 | $CH_3$ | $CH_3$ |
| 3-F,4-$CH_3$ | 3 | 2 | 2 | $CH_3$ | H |
| 3-F,4-$CH_3$ | 3 | 2 | 2 | $CH_3$ | $CH_3$ |

e) Préparation des dérivés de formule générale II :

10,48g (0,0278 mole) de [(p.fluorophényl-3 propionyl)-5 thiényl-2]-5 diméthyl-3,3 pentanoate de méthyle sont mis en solution dans 80ml de triéthylèneglycol porté à 70 °C. 3,12g (0,0974 mole) d'hydrate d'hydrazine à 80% sont ajoutés.

La température est portée à 100 °C et 4,68g (0,083 mole) de potasse en pastilles sont ajoutées en une fois. Après 30 min de reflux, l'eau formée est distillée en une heure durant laquelle la température monte à 210-220 °C. Après une heure à cette température la réaction est terminée.

Le milieu réactionnel est hydrolysé, après refroidissement à 20 °C, par 200ml d'eau, acidifié par 30ml d'HCl concentré et extrait par 3 fois 100ml d'éther sulfurique. Les phases éthérées réunies sont lavées par une solution normale d'HCl puis de l'eau, traitées par Mg $SO_4$ anhydre puis le noir animal et concentrées à sec.

Elles fournissent 10,5g d'huile brute qui est chromatographiée ($SiO_2/CH_2Cl_2$) pour donner 9,10g d'une huile incolore. Rendement : 94%.

Ont ainsi été préparés les produits regroupés dans le tableau 9 ci-après.

**Tableau 9** : Dérivés de formule :

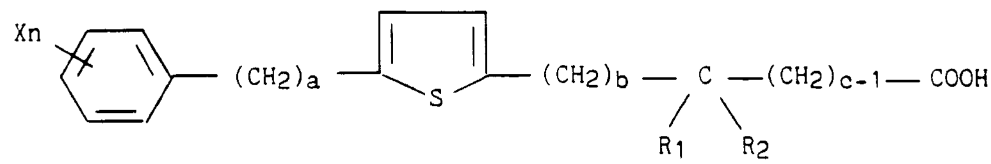

| X | a | b | c | $R_1$ | $R_2$ |
|---|---|---|---|---|---|
| H | 3 | 2 | 2 | $CH_3$ | $CH_3$ |
| p.F | 3 | 2 | 2 | $CH_3$ | $CH_3$ |
| p.F | 3 | 2 | 2 | $CH_3$ | H |
| m.F | 3 | 2 | 2 | $CH_3$ | $CH_3$ |
| o.Cl | 3 | 2 | 2 | $CH_3$ | $CH_3$ |
| H | 6 | 2 | 2 | $CH_3$ | $CH_3$ |
| 2,4-di$CH_3$ | 3 | 2 | 2 | $CH_3$ | H |
| 2,4-di$CH_3$ | 3 | 2 | 2 | $CH_3$ | $CH_3$ |
| m.F | 3 | 2 | 2 | $CH_3$ | H |
| p.$CH_3$ | 4 | 2 | 2 | $CH_3$ | H |
| p.$CH_3$ | 4 | 2 | 2 | $CH_3$ | $CH_3$ |
| 2,5-di$CH_3$ | 3 | 2 | 2 | $CH_3$ | $CH_3$ |
| 2,5-di$CH_3$ | 3 | 2 | 2 | $CH_3$ | H |
| p.$CH_3$ | 3 | 2 | 2 | H | H |
| p.$CH_3$ | 3 | 1 | 3 | $CH_3$ | $CH_3$ |
| p.$CH_3$ | 3 | 3 | 1 | $CH_3$ | $CH_3$ |
| p.-$CH(CH_3)_2$ | 3 | 2 | 2 | $CH_3$ | H |
| p.-$CH(CH_3)_2$ | 3 | 2 | 2 | $CH_3$ | $CH_3$ |
| p.-$(CH_2)_4$-$CH_3$ | 3 | 2 | 2 | $CH_3$ | H |
| p.-$(CH_2)_4$-$CH_3$ | 3 | 2 | 2 | $CH_3$ | $CH_3$ |
| 3-F, 4-$CH_3$ | 3 | 2 | 2 | $CH_3$ | H |
| 3-F, 4-$CH_3$ | 3 | 2 | 2 | $CH_3$ | $CH_3$ |

**II) Synthèse des dérivés de formule générale I :**

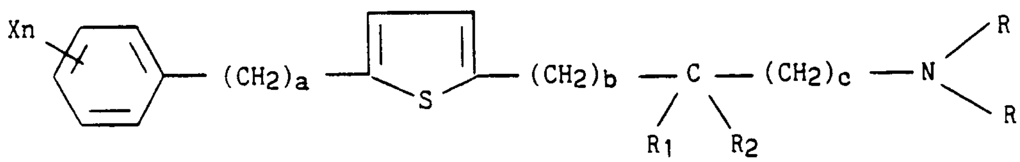

(I)

**A) Préparation des dérivés de formule générale V :** 16,5g (0,05 mole) de (phényl-3 propyl)-5 (carboxy-4 diméthyl-3,3 butyl)-2 thiophène et 6,5g (0,055 mole) de chlorure de thionyle sont mis en solution dans 300ml de chloroforme anhydre, et l'agitation est maintenue jusqu'à la fin du dégagement gazeux. Puis, le solvant et l'excès de chlorure de thionyle sont distillés, sous pression réduite.

Le chlorure d'acide ainsi obtenu, en solution dans 250ml d'éther anhydre, et 8,71g (0,1 mole) de morpholine en solution dans 150ml d'éther anhydre sont coulés simultanément sur 100ml d'éther anhydre agités magnétiquement.

Après 20 minutes, le chlorhydrate de morpholinium formé est filtré et lavé à l'éther.

La phase éthérée est lavée par 2 fois 30ml d'eau, séchée sur Mg $SO_4$ et décolorée au noir animal.

L'évaporation du solvant laisse 18,8g d'une huile incolore : le (phényl-3 propyl)-5 (diméthyl-3,3 N-morpholinocarbonyl-4 butyl)-2 thiophène.

Ont ainsi été préparés les produits regroupés dans le tableau 10 ci-après.

**Tableau 10** : Dérivés de formule :

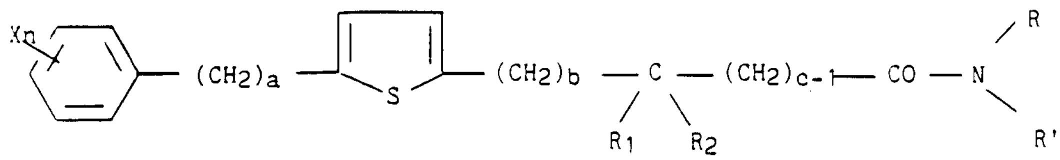

| Xn | a | b | c | $R_1$ | $R_2$ | 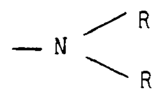 |
|---|---|---|---|---|---|---|
| H | 2 | 2 | 2 | $CH_3$ | $CH_3$ | 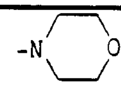 |
| p.$CH_3$ | 2 | 2 | 2 | $CH_3$ | H | 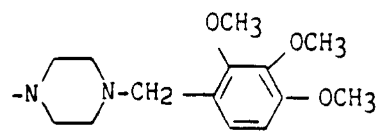 |
| p.$CH_3$ | 2 | 2 | 2 | $CH_3$ | $CH_3$ | 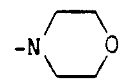 |
| p.$CH_3$ | 2 | 2 | 2 | $CH_3$ | $CH_3$ | 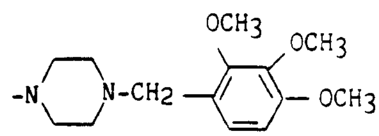 |
| H | 3 | 2 | 2 | H | H | 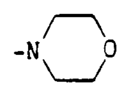 |
| p.$CH_3$ | 3 | 2 | 2 | H | H | 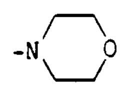 |
| H | 3 | 2 | 2 | $CH_3$ | H | 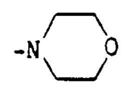 |
| p.$CH_3$ | 3 | 2 | 2 | $CH_3$ | H | 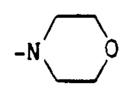 |
| p.F | 3 | 2 | 2 | $CH_3$ | H | 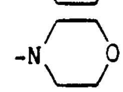 |
| H | 3 | 2 | 2 | $CH_3$ | $CH_3$ | 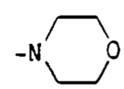 |
| p.$CH_3$ | 3 | 2 | 2 | $CH_3$ | $CH_3$ | 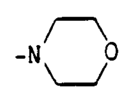 |
| m.$CH_3$ | 3 | 2 | 2 | $CH_3$ | $CH_3$ | 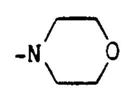 |

| Xn | a | b | c | $R_1$ | $R_2$ | 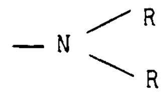 |
|---|---|---|---|---|---|---|
| p.F | 3 | 2 | 2 | $CH_3$ | $CH_3$ | 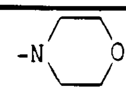 |
| m.F | 3 | 2 | 2 | $CH_3$ | $CH_3$ | 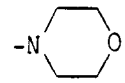 |
| 2,4 di$CH_3$ | 3 | 2 | 2 | $CH_3$ | H | 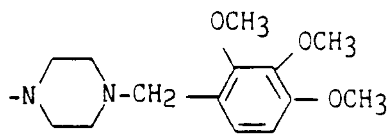 |
| 2,4 di$CH_3$ | 3 | 2 | 2 | $CH_3$ | $CH_3$ | 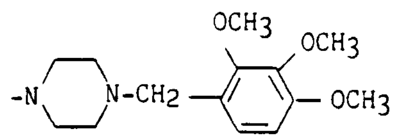 |
| 2,5 di$CH_3$ | 3 | 2 | 2 | $CH_3$ | H | 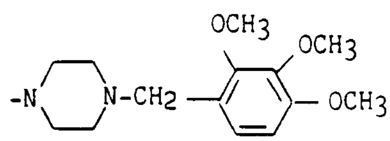 |
| 2,5 di$CH_3$ | 3 | 2 | 2 | $CH_3$ | $CH_3$ | 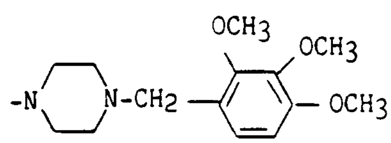 |
| p.$CH_3$ | 4 | 2 | 2 | $CH_3$ | H | 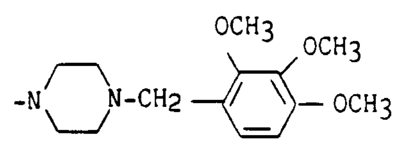 |
| p.$CH_3$ | 4 | 2 | 2 | $CH_3$ | $CH_3$ | 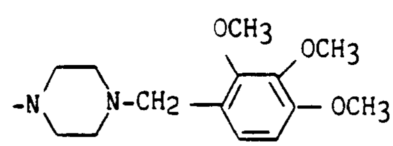 |

| Xn | a | b | c | $R_1$ | $R_2$ | 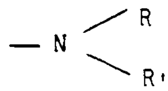 |
|---|---|---|---|---|---|---|
| O.cl | 3 | 2 | 2 | $CH_3$ | $CH_3$ | 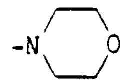 |
| H | 3 | 2 | 2 | 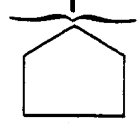 | | 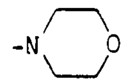 |
| H | 3 | 2 | 2 | $CH_3$ | $CH_3$ | 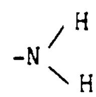 |
| H | 3 | 2 | 2 | $CH_3$ | $CH_3$ | 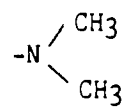 |
| H | 3 | 2 | 2 | $CH_3$ | $CH_3$ | 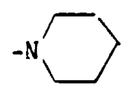 |
| H | 3 | 2 | 2 | $CH_3$ | $CH_3$ | 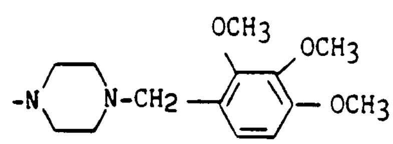 |
| m.F | 3 | 2 | 2 | $CH_3$ | $CH_3$ | 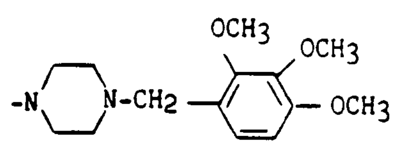 |

| Xn | a | b | c | $R_1$ | $R_2$ | 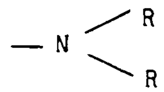 |
|---|---|---|---|---|---|---|
| m.F | 3 | 2 | 2 | $CH_3$ | H | 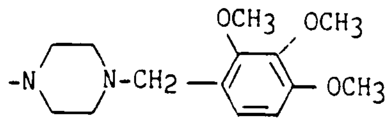 |
| p.F | 3 | 2 | 2 | $CH_3$ | $CH_3$ | 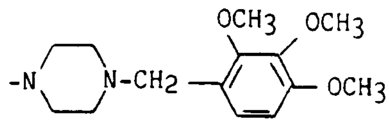 |
| p-$CH_3$ | 3 | 2 | 2 | $CH_3$ | $CH_3$ | 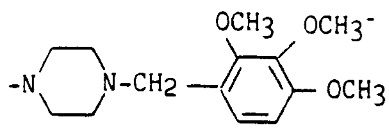 |
| H | 3 | 3 | 1 | $CH_3$ | $CH_3$ | 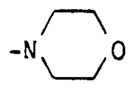 |
| p.$CH_3$ | 3 | 3 | 1 | $CH_3$ | $CH_3$ | 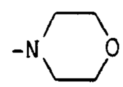 |
| H | 4 | 2 | 2 | $CH_3$ | $CH_3$ | 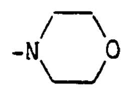 |
| p.$CH_3$ | 4 | 2 | 2 | $CH_3$ | $CH_3$ | 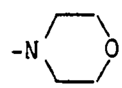 |
| H | 6 | 2 | 2 | $CH_3$ | $CH_3$ | 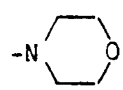 |
| H | 6 | 2 | 2 | $CH_3$ | $CH_3$ | 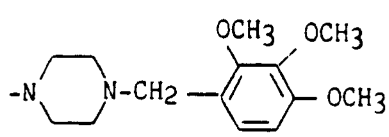 |

| Xn | a | b | c | $R_1$ | $R_2$ | 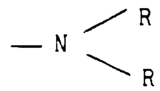 |
|---|---|---|---|---|---|---|
| p.$CH_3$ | 3 | 2 | 2 | H | H | 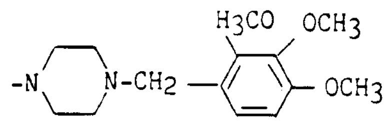 |
| p.$CH_3$ | 3 | 1 | 3 | $CH_3$ | $CH_3$ | " |
| p.$CH_3$ | 3 | 3 | 1 | $CH_3$ | $CH_3$ | " |
| p.$CH_3$ | 3 | 1 | 3 | $CH_3$ | $CH_3$ | 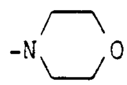 |
| p.-$CH(CH_3)_2$ | 3 | 2 | 2 | $CH_3$ | H | 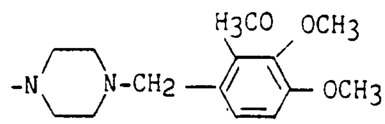 |
| p.-$CH(CH_3)_2$ | 3 | 2 | 2 | $CH_3$ | $CH_3$ | " |
| p.-$CH(CH_3)_2$ | 3 | 2 | 2 | $CH_3$ | H | 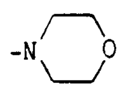 |
| p.-$CH(CH_3)_2$ | 3 | 2 | 2 | $CH_3$ | $CH_3$ | " |
| 3-F, 4-$CH_3$ | 3 | 2 | 2 | $CH_3$ | H | 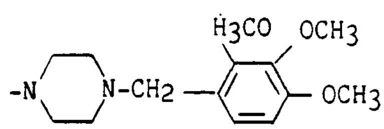 |
| 3-F, 4-$CH_3$ | 3 | 2 | 2 | $CH_3$ | $CH_3$ | " |
| p.-$(CH_2)_4$-$CH_3$ | 3 | 2 | 2 | $CH_3$ | H | 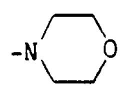 |
| p.-$(CH_2)_4$-$CH_3$ | 3 | 2 | 2 | $CH_3$ | $CH_3$ | " |
| p.-$(CH_2)_4$-$CH_3$ | 3 | 2 | 2 | $CH_3$ | H | 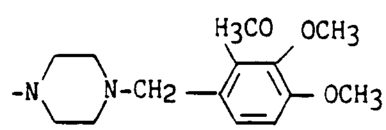 |
| p.-$(CH_2)_4$-$CH_3$ | 3 | 2 | 2 | $CH_3$ | $CH_3$ | " |

**B) Préparation des dérivés de formule générale I :**

18g (0,045 mole) de (phényl-3 propyl)-5 (diméthyl-3,3 N-morpholinocarbonyl-4 butyl)-2 thiophène en solution dans 150ml d'éther sont versés, goutte à goutte, sur une suspension de 4g (0,1 mole)) d'hydrure de lithium aluminium dans 200ml d'éther au reflux.

Le reflux est maintenu 2 heures après l'addition, puis le mélange réactionnel est refroidi à 20 °C et hydrolysé avec précaution par 4ml d'eau, 4ml de soude aqueuse 4N, puis 8ml d'eau. Le solide formé est filtré et lavé à l'éther.

La phase éthérée est lavée par 3 fois 50ml d'eau, séchée sur Mg $SO_4$, décolorée au noir animal et concentrée à sec.

Le résidu est chromatographié sur $SiO_2$ (solvant : $CH_2$ $Cl_2$ - $CH_3$ COO $C_2H_5$, 95-5).

Les fractions contenant l'amine sont concentrées à sec. Le résidu est repris dans 600ml d'éther anhydre et le chlorhydrate est obtenu par addition d'un équivalent d'HCl en solution dans l'éther.

Après une heure d'agitation, le chlorhydrate est filtré, lavé à l'éther, essoré et séché sous vide. On obtient 16g de chlorhydrate de (phényl-3 propyl)-5 (diméthyl-3,3 N-morpholino-5 pentyl)-2 thiophène, sous forme d'un solide blanc (cf exemple8 du tableau 11 ci-après).

Ont ainsi été préparés les produits correspondants aux exemples 1 à 51 regroupés dans le tableau 11 ci-après.

**Tableau 11 : Dérivés de formule :**

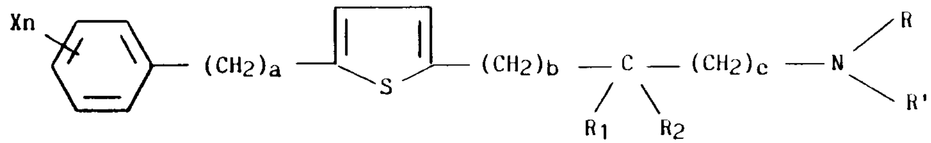 (I)

| Exemples n° | X | n | a | b | c | $R_1$ | $R_2$ | 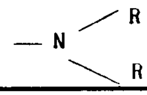 | forme isolée | P.F. °C (Kofler) |
|---|---|---|---|---|---|---|---|---|---|---|
| 1 | H | 1 | 2 | 2 | 2 | $CH_3$ | $CH_3$ | 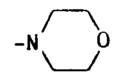 | HCl | 210 |
| 2 | p.$CH_3$ | 1 | 2 | 2 | 2 | $CH_3$ | $CH_3$ | 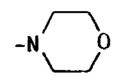 | HCl | 220-222 |
| 3 | H | 1 | 3 | 2 | 2 | H | H | 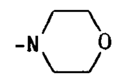 | $CH_3SO_3H$ | 81 |
| 4 | p.$CH_3$ | 1 | 3 | 2 | 2 | H | H | 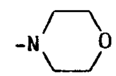 | HCl | 112-113 |
| 5 | H | 1 | 3 | 2 | 2 | $CH_3$ | H | 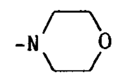 | HCl | 146-148 |
| 6 | p.$CH_3$ | 1 | 3 | 2 | 2 | $CH_3$ | H | 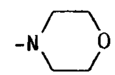 | HCl | 150-152 |
| 7 | p.F | 1 | 3 | 2 | 2 | $CH_3$ | H | 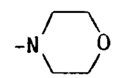 | HCl | 146 |
| 8 | H | 1 | 3 | 2 | 2 | $CH_3$ | $CH_3$ | 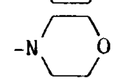 | $CH_3SO_3H$ | ≃ 100 |
| 9 | p.$CH_3$ | 1 | 3 | 2 | 2 | $CH_3$ | $CH_3$ | 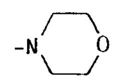 | HCl | 207-208 |

**Tableau 11 suite 1**

| Exemples n° | X | n | a | b | c | R1 | R2 | 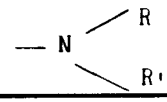 | forme isolée | P.F. °C (Kofler) |
|---|---|---|---|---|---|---|---|---|---|---|
| 10 | m.$CH_3$ | 1 | 3 | 2 | 2 | $CH_3$ | $CH_3$ | 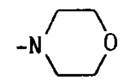 | HCl | ≃206 |
| 11 | p.F | 1 | 3 | 2 | 2 | $CH_3$ | $CH_3$ | 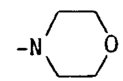 | HCl | 195-200 |
| 12 | m.F | 1 | 3 | 2 | 2 | $CH_3$ | $CH_3$ | 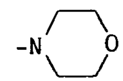 | HCl | 190 |
| 13 | o.Cl | 1 | 3 | 2 | 2 | $CH_3$ | $CH_3$ | 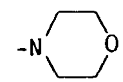 | HCl | 205 |
| 14 | H | 1 | 3 | 2 | 2 | 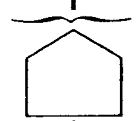 | | 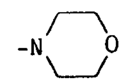 | HCl | 166-168 |
| 15 | H | 1 | 3 | 2 | 2 | $CH_3$ | $CH_3$ | 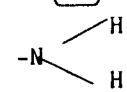 | HCl | 108 |
| 16 | H | 1 | 3 | 2 | 2 | $CH_3$ | $CH_3$ | 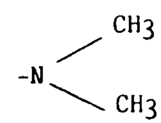 | HCl | 170 |
| 17 | H | 1 | 3 | 2 | 2 | $CH_3$ | $CH_3$ | 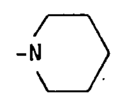 | HCl | 110-112 |

**Tableau 11 suite 2**

| Exemples n° | X | n | a | b | c | $R_1$ | $R_2$ | 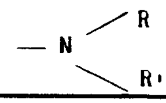 | forme isolée | P.F. °C (Kofler) |
|---|---|---|---|---|---|---|---|---|---|---|
| 18 | H | 1 | 3 | 2 | 2 | $CH_3$ | $CH_3$ | 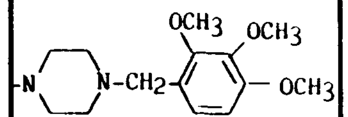 | 2 HCl | 206 |
| 19 | m.F | 1 | 3 | 2 | 2 | $CH_3$ | $CH_3$ | 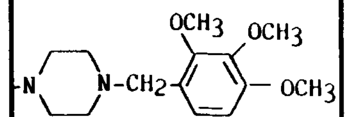 | 2 HCl | 207 |
| 20 | p.F | 1 | 3 | 2 | 2 | $CH_3$ | $CH_3$ | 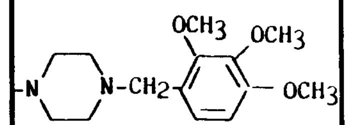 | 2 HCl | 235 |
| 21 | H | 1 | 3 | 3 | 1 | $CH_3$ | $CH_3$ | 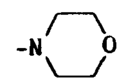 | HCl | 108-110 |
| 22 | p.$CH_3$ | 1 | 3 | 3 | 1 | $CH_3$ | $CH_3$ | 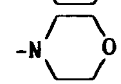 | HCl | 128-130 |
| 23 | H | 1 | 4 | 2 | 2 | $CH_3$ | $CH_3$ | 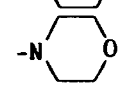 | HCl | 194-196 |
| 24 | p.$CH_3$ | 1 | 4 | 2 | 2 | $CH_3$ | $CH_3$ | 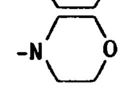 | HCl | 192-194 |
| 25 | H | 1 | 6 | 2 | 2 | $CH_3$ | $CH_3$ | 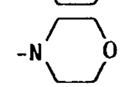 | HCl | 197 |

Tableau 11 suite 3

| Exemples n° | X | n | a | b | c | $R_1$ | $R_2$ | 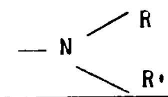 | forme isolée | P.F. °C (capillaire) |
|---|---|---|---|---|---|---|---|---|---|---|
| 26 | p.$CH_3$ | 1 | 3 | 2 | 2 | $CH_3$ | $CH_3$ | 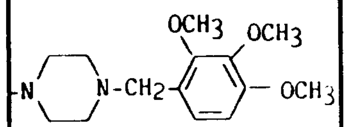 | 2 HCl | 210 |
| 27 | m.F | 1 | 3 | 2 | 2 | $CH_3$ | H | " | 2 HCl | 190 |
| 28 | p.$CH_3$ | 1 | 3 | 2 | 2 | $CH_3$ | H | " | 2 HCl | 188-190 |
| 29 | p.$CH_3$ | 1 | 2 | 2 | 2 | $CH_3$ | H | " | 2 HCl | 198-202 |
| 30 | p.$CH_3$ | 1 | 2 | 2 | 2 | $CH_3$ | $CH_3$ | " | 2 HCl | 225-230 |
| 31 | H | 1 | 6 | 2 | 2 | $CH_3$ | $CH_3$ | " | 2 HCl | > 240 (dec) |
| 32 | 2,4 di$CH_3$ | 2 | 3 | 2 | 2 | $CH_3$ | H | " | 2 HCl | 230-235 |
| 33 | 2,4 di$CH_3$ | 2 | 3 | 2 | 2 | $CH_3$ | $CH_3$ | " | 2 HCl | 235 |
| 34 | 3,5 di$CH_3$ | 2 | 3 | 2 | 2 | $CH_3$ | H | " | | |
| 35 | 3,5 di$CH_3$ | 2 | 3 | 2 | 2 | $CH_3$ | $CH_3$ | " | | |
| 36 | p.$CH_3$ | 1 | 4 | 2 | 2 | $CH_3$ | H | " | 2 HCl | 227- 230 |

Tableau 11 suite 4

| Exemples n° | X | n | a | b | c | $R_1$ | $R_2$ | 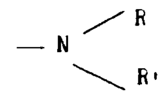 | forme isolée | P.F. °C (capillaire) |
|---|---|---|---|---|---|---|---|---|---|---|
| 37 | p.$CH_3$ | 1 | 4 | 2 | 2 | $CH_3$ | $CH_3$ | 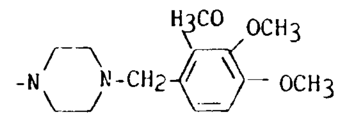 | 2 HCl | 238-240 |
| 38 | p.$CH_3$ | 1 | 3 | 2 | 2 | H | H | " | 2 HCl | 212-214 |
| 39 | p.$CH_3$ | 1 | 3 | 1 | 3 | $CH_3$ | $CH_3$ | " | 2 HCl | 225-230 (dec) |
| 40 | p.$CH_3$ | 1 | 3 | 3 | 1 | $CH_3$ | $CH_3$ | " | | |
| 41 | p.$CH_3$ | 1 | 3 | 1 | 3 | $CH_3$ | $CH_3$ | 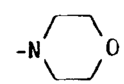 | | |
| 42 | p.-$CH(CH_3)_2$ | 1 | 3 | 2 | 2 | $CH_3$ | H | 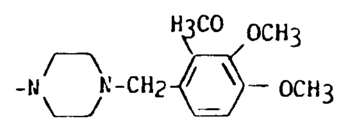 | | |
| 43 | p.-$CH(CH_3)_2$ | 1 | 3 | 2 | 2 | $CH_3$ | $CH_3$ | " | | |
| 44 | p.-$CH(CH_3)_2$ | 1 | 3 | 2 | 2 | $CH_3$ | H | 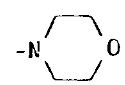 | HCl | 170 |
| 45 | p.-$CH(CH_3)_2$ | 1 | 3 | 2 | 2 | $CH_3$ | $CH_3$ | " | HCl | 210 |

Tableau 11 suite 5

| Exemples n° | X | n | a | b | c | $R_1$ | $R_2$ | 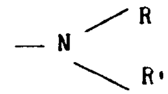 | forme isolée | P.F. °C (capillaire) |
|---|---|---|---|---|---|---|---|---|---|---|
| 46 | 3-F,4-$CH_3$ | 2 | 3 | 2 | 2 | $CH_3$ | H | 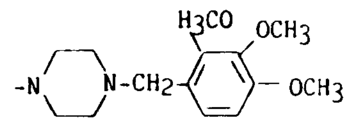 | 2 HCl | 195-198 |
| 47 | 3-F,4-$CH_3$ | 2 | 3 | 2 | 2 | $CH_3$ | $CH_3$ | " | | |
| 48 | p.-$(CH_2)_4$-$CH_3$ | 1 | 3 | 2 | 2 | $CH_3$ | H | 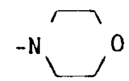 | | |
| 49 | p.-$(CH_2)_4$-$CH_3$ | 1 | 3 | 2 | 2 | $CH_3$ | $CH_3$ | " | | |
| 50 | p.-$(CH_2)_4$-$CH_3$ | 1 | 3 | 2 | 2 | $CH_3$ | H | 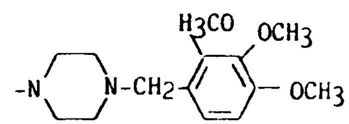 | 2 HCl | 220-225 (dec) |
| 51 | p.-$(CH_2)_4$-$CH_3$ | 1 | 3 | 2 | 2 | $CH_3$ | $CH_3$ | " | 2 HCl | > 240 (dec) |

**Revendications**

**Revendications pour les Etats contractants suivants : AT, BE, CH, DE, DK, FR, GB, IT, LI, LU, NL, SE**

**1.** Les dérivés du thiophène de formule générale I :

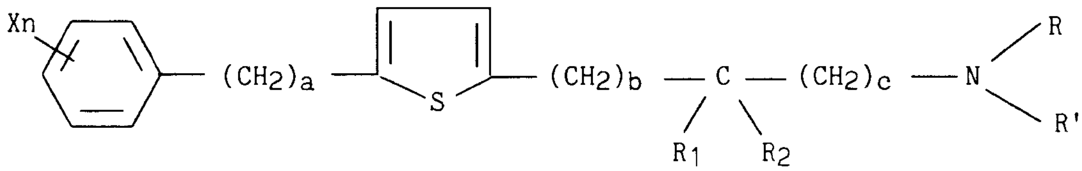

(I)

dans laquelle :

- X représente un atome d'hydrogène ou d'halogène, un radical alkyle ou alkoxy ayant chacun de 1 à 5 atomes de carbone en chaîne droite ou ramifiée ou un radical dialkylamino dans lequel chaque groupe alkyle renferme de 1 à 5 atomes de carbone ;
- n représente 1 ou 2 ;
- a représente un nombre entier de 2 à 6 inclus ;
- b représente 2 ou 3 ;
- c représente 1 ou 2, de telle sorte que b + c = 4 ;
- $R_1$ et $R_2$, identiques ou différents, représentent chacun un atome d'hydrogène ou un radical alkyle ayant de 1 à 5 atomes de carbone, ou
  $R_1$ et $R_2$, forment ensemble avec l'atome de carbone auquel ils sont liés un cycle hydrocarboné renfermant de 3 à 6 atomes de carbone ; et
- R et R', identiques ou différents, représentent chacun un atome d'hydrogène ou un radical alkyle ayant de 1 à 5 atomes de carbone, ou
  R et R' forment ensemble avec l'atome d'azote auquel ils sont liés un radical hétérocyclique penta ou hexagonal renfermant éventuellement un atome d'oxygène ou un deuxième atome d'azote, lequel peut être lui-même substitué par un radical alkyle renfermant de 1 à 5 atomes de carbone, ou par un radical arylalkyle dans lequel le groupe alkyle renferme de 1 à 5 atomes de carbone et le groupe aryle est soit non substitué soit mono- ou poly- substitué par un atome d'halogène ou un radical alkyle ou alkyloxy ayant chacun de 1 à 5 atomes de carbone,

et leurs sels d'addition physiologiquement tolérables avec des acides appropriés.

**2.** Les composés de la revendication 1 qui sont :
le (phényl-2 éthyl)-5 (N-morpholino-5 diméthyl-3,3 pentyl)-2 thiophène, et son chlorhydrate,
le (p.méthylphényl-2 éthyl)-5 (N-morpholino-5 diméthyl-3,3 pentyl)-2 thiophène, et son chlorhydrate,
le (phényl-3 propyl)-5 (N-morpholino-5 pentyl)-2 thiophène, et son méthanesulfonate,
le (p.méthylphényl-3 propyl)-5 (N-morpholino-5 pentyl)-2 thiophène, et son chlorhydrate,
le (phényl-3 propyl)-5 (N- morpholino-5 méthyl-3 pentyl)-2 thiophène, et son chlorhydrate,
le (p.méthylphényl-3 propyl)-5 (N-morpholino-5 méthyl-3 pentyl)-2 thiophène, et son chlorhydrate,
le (p.fluorophényl-3 propyl)-5 (N-morpholino-5 méthyl-3 pentyl)-2 thiophène et son chlorhydrate,
le (phényl-3 propyl)-5 (N-morpholino-5 diméthyl-3,3 pentyl)-2 thiophène, et son méthanesulfonate,
le (p.méthylphényl-3 propyl)-5 (N-morpholino-5 diméthyl-3,3 pentyl)-2 thiophène et son chlorhydrate,
le (p.fluorophényl-3 propyl)-5 (N-morpholino-5 diméthyl-3,3 pentyl)-2 thiophène, et son chlorhydrate,
le (m.fluorophényl-3 propyl)-5 (N-morpholino-5 diméthyl-3,3 pentyl)-2 thiophène, et son chlorhydrate,
l'(o.chlorophényl-3 propyl)-5 (N-morpholino-5 diméthyl-3,3 pentyl)-2 thiophène, et son chlorhydrate,
le (phényl-3 propyl)-5 (N-morpholino-5 tétraméthylène-3,3 pentyl)-2 thiophène, et son chlorhydrate,
le (p.méthylphényl-3 propyl)-5 (N-morpholino-5 diméthyl-4,4 pentyl)-2 thiophène, et son chlorhydrate.

**3.** Les composés de la revendication 1 qui sont :
le (p.fluorophényl-3 propyl)-5 {[(triméthoxy-2,3,4 benzyl)-4 pipérazinyl]-5 diméthyl-3,3 pentyl}-2 thiophène, et son dichlorhydrate,
le (phényl-3 propyl)-5 {[(triméthoxy-2,3,4 benzyl)-4 pipérazinyl]-5 diméthyl-3,3 pentyl}-2 thiophène, et son dichlorhydrate,
le (m.fluorophényl-3 propyl)-5 {[(triméthoxy-2,3,4 benzyl)-4 pipérazinyl]-5 diméthyl-3,3 pentyl}-2 thiophène, et son dichlorhydrate,

le (p.méthylphényl-3 propyl)-5 {[(triméthoxy-2,3,4 benzyl)-4 pipérazinyl]-5 diméthyl-3,3 pentyl}-2 thiophène, et son dichlorhydrate,
le (m.fluorophényl-3 propyl)-5 {[(triméthoxy-2,3,4 benzyl)-4 pipérazinyl]-5 méthyl-3 pentyl}-2 thiophène,
le (p.méthylphényl-2 éthyl)-5 {[(triméthoxy-2,3,4 benzyl)-4 pipérazinyl]-5 méthyl-3 pentyl}-2 thiophène,
le (p.méthylphényl-2 éthyl)-5 {[(triméthoxy-2,3,4 benzyl)-4 pipérazinyl]-5 diméthyl-3,3 pentyl}-2 thiophène,
le (phényl-6 hexyl)-5 {[(triméthoxy-2,3,4 benzyl)-4 pipérazinyl]-5 diméthyl-3,3 pentyl}-2 thiophène,
le [(diméthyl-2,4 phényl)-3 propyl]-5 {[(triméthoxy-2,3,4 benzyl)-4 pipérazinyl]-5 méthyl-3 pentyl}-2 thiophène,
le [(diméthyl-2,4 phényl-3 propyl]-5 {[(triméthoxy-2,3,4 benzyl)-4 pipérazinyl]-5 diméthyl-3,3 pentyl}-2 thiophène,
le [(diméthyl-3,5 phényl)-3 propyl]-5 {[(triméthoxy-2,3,4 benzyl)-4 pipérazinyl]-5 méthyl-3 pentyl}-2 thiophène,
le [(diméthyl-3,5 phenyl)-3 propyl]-5 {[(triméthoxy-2,3,4 benzyl)-4 pipérazinyl]-5 diméthyl-3,3 pentyl}-2 thiophène,
le (p.méthylphényl-4 butyl)-5 {[(triméthoxy-2,3,4 benzyl)-4 pipérazinyl]-5 méthyl-3 pentyl}-2 thiophène,
le (p.méthylphényl-4 butyl)-5 {[(triméthoxy-2,3,4 benzyl)-4 pipérazinyl]-5 diméthyl-3,3 pentyl}-2 thiophène.

**4.** Le procédé de préparation des dérivés de la revendication 1,
caractérisé en ce que :
l'on transforme, l'acide de formule générale II :

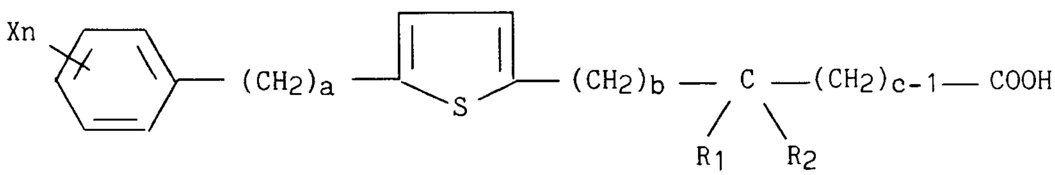

(II)

dans laquelle X, n, a, b, c, $R_1$ et $R_2$ ont les significations définies, dans la revendication 1, en chlorure d'acide correspondant de formule générale III :

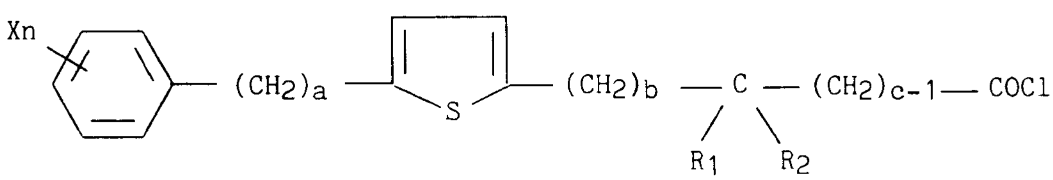

(III)

dans laquelle X, n, a, b, c, $R_1$ et $R_2$ ont les significations définies, dans la revendication 1,
lequel chlorure d'acide sert à acyler l'amine de formule générale IV :

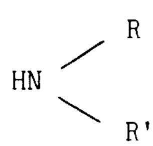

(IV)

dans laquelle R et R' ont les significations définies dans la revendication 1,
et l'on réduit l'amide ainsi obtenue de formule générale V :

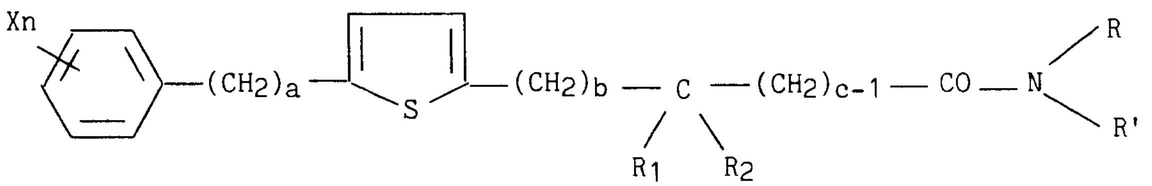

(V)

dans laquelle X, n, a, b, c, $R_1$, $R_2$, R et R' ont les significations définies dans la revendication 1.

**5.** Les compositions pharmaceutiques contenant comme principe actif un dérivé selon les revendications 1 à 3, avec des excipients pharmaceutiques appropriés.

**6.** Les compositions pharmaceutiques selon la revendication 5, présentées sous une forme convenant notamment pour le traitement des pathologies caractérisées par une perte du tissu osseux.

**7.** A titre de produits industriels nouveaux, utilisables dans la synthèse des dérivés de formule I selon la revendication 1, les amides de formule générale V :

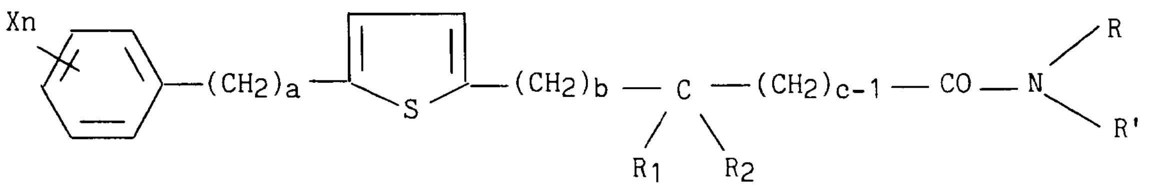

(V)

dans laquelle X, n, a, b, c, $R_1$, $R_2$, R et R' ont les significations définies dans la revendication 1.

**Revendications pour les Etats contractants suivants : ES, GR**

**1.** Le procédé de préparation des dérivés du thiophène de formule générale I :

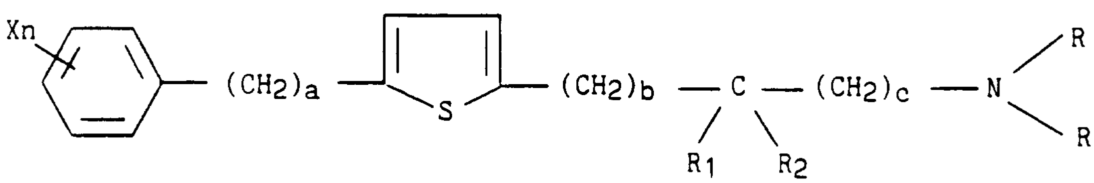

(I)

dans laquelle :

- X représente un atome d'hydrogène ou d'halogène, un radical alkyle ou alkoxy ayant chacun de 1 à 5 atomes de carbone en chaîne droite ou ramifiée ou un radical dialkylamino dans lequel chaque groupe alkyle renferme de 1 à 5 atomes de carbone ;
- n représente 1 ou 2 ;
- a représente un nombre entier de 2 à 6 inclus ;
- b représente 2 ou 3 ;
- c représente 1 ou 2, de telle sorte que b + c = 4 ;
- $R_1$ et $R_2$, identiques ou différents, représentent chacun un atome d'hydrogène ou un radical alkyle ayant de 1 à 5 atomes de carbone, ou
  $R_1$ et $R_2$, forment ensemble avec l'atome de carbone auquel ils sont liés un cycle hydrocarboné renfermant de 3 à 6 atomes de carbone ; et
- R et R', identiques ou différents, représentent chacun un atome d'hydrogène ou un radical alkyle ayant de 1 à 5 atomes de carbone, ou

- R et R' forment ensemble avec l'atome d'azote auquel ils sont liés un radical hétérocyclique penta ou hexagonal renfermant éventuellement un atome d'oxygène ou un deuxième atome d'azote, lequel peut être lui-même substitué par un radical alkyle renfermant de 1 à 5 atomes de carbone, ou par un radical arylalkyle dans lequel le groupe alkyle renferme de 1 à 5 atomes de carbone et le groupe aryle est soit non substitué soit mono- ou poly- substitué par un atome d'halogène ou un radical alkyle ou alkyloxy ayant chacun de 1 à 5 atomes de carbone ;

et de leurs sels physiologiquement tolérables avec des acides appropriés ;
caractérisé en ce que :

- l'on transforme, l'acide de formule générale II :

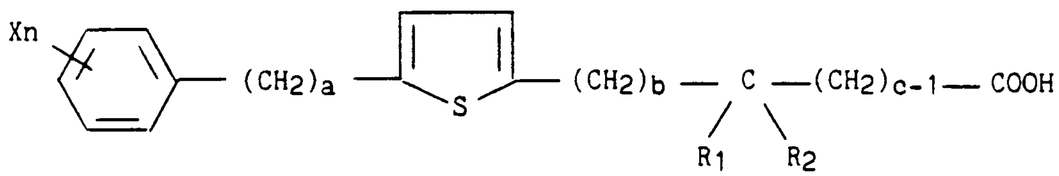
(II)

dans laquelle X, n, a, b, c, $R_1$ et $R_2$ ont les significations définies précédemment,
en chlorure d'acide correspondant de formule générale III :

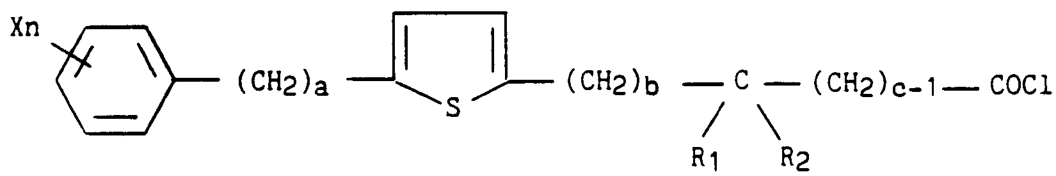
(III)

dans laquelle X, n, a, b, c, $R_1$ et $R_2$ ont les significations définies précédemment,
lequel chlorure d'acide sert à acyler l'amine de formule générale IV :

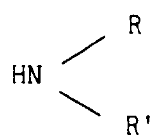
(IV)

dans laquelle R et R' ont les significations définies précédemment,
et l'on réduit l'amide ainsi obtenue de formule générale V :

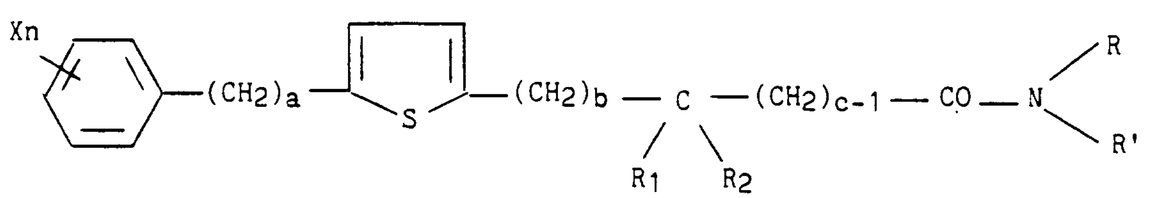
(V)

dans laquelle X, n, a, b, c, $R_1$, $R_2$, R et R' ont les signification définies précédemment ;
et, si on le désire, on traite les dérivés ainsi obtenus avec des acide appropriés, pour donner les sels d'addition correspondants.

**2.** Le procédé de préparation des intermédiaires de synthèse des dérivés de formule générale I, lesquels intermédiaires répondent à la formul générale V :

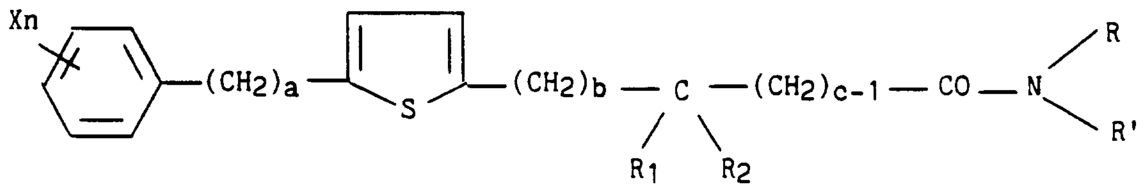

(V)

dans laquelle X, n, a, b, c, $R_1$,$R_2$, R et R' ont les significations définies dans la revendication 1,

- caractérisé en ce que l'on opère comme inclus dans la revendication 1 à partir du dérivé II jusqu'à l'obtention des amides V.

## Claims

### Claims for the following Contracting States : AT, BE, CH, DE, DK, FR, GB, IT, LI, LU, NL, SE

**1.** Thiophene compounds of the general formula I :

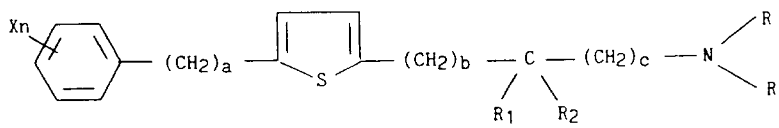

(I)

in which :

- X represents a hydrogen or halogen atom, a straight-chain or branched alkyl or alkoxy radical each having from 1 to 5 carbon atoms, or a dialkylamino radical in which each alkyl group contains from 1 to 5 carbon atoms;
- n represents 1 or 2;
- a represents an integer of from 2 to 6 inclusive;
- b represents 2 or 3;
- c represents 1 or 2, and is such that b + c = 4;
- $R_1$ and $R_2$, which may be the same or different, each represents a hydrogen atom or an alkyl radical having from 1 to 5 carbon atoms, or
  $R_1$ and $R_2$ together with the carbon atom to which they are bonded form a hydrocarbon ring containing from 3 to 6 carbon atoms; and
- R and R', which may be the same or different, each represents a hydrogen atom or an alkyl radical having from 1 to 5 carbon atoms, or
  R and R' together with the nitrogen atom to which they are bonded form a pentagonal or hexagonal heterocyclic radical optionally containing an oxygen atom or a second nitrogen atom which may itself be substituted by an alkyl radical containing from 1 to 5 carbon atoms or by an arylalkyl radical in which the alkyl group contains from 1 to 5 carbon atoms and the aryl group is either unsubstituted or mono- or poly-substituted by a halogen atom or an alkyl or alkoxy radical each having from 1 to 5 carbon atoms,

and the physiologically tolerable addition salts thereof with appropriate acids.

**2.** The compounds of claim 1 which are:
5-(2-phenylethyl)-2-(5-N-morpholino-3,3-dimethylpentyl)-thiophene and its hydrochloride,
5-(2-p-methylphenylethyl)-2-(5-N-morpholino-3,3-dimethylpentyl)-thiophene and its hydrochloride,
5-(3-phenylpropyl)-2-(5-N-morpholinopentyl)-thiophene and its methanesulphonate,
5-(3-p-methylphenylpropyl)-2-(5-N-morpholinopentyl)-thiophene and its hydrochloride,
5-(3-phenylpropyl)-2-(5-N-morpholino-3-methylpentyl)-thiophene and its hydrochloride,
5-(3-p-methylphenylpropyl)-2-(5-N-morpholino-3-methylpentyl)-thiophene and its hydrochloride,
5-(3-p-fluorophenylpropyl)-2-(5-N-morpholino-3-methylpentyl)-thiophene and its hydrochloride,
5-(3-phenylpropyl)-2-(5-N-morpholino-3,3-dimethylpentyl)-thiophene and its methanesulphonate,
5-(3-p-methylphenylpropyl)-2-(5-N-morpholino-3,3-dimethylpentyl)-thiophene and its hydrochloride,
5-(3-p-fluorophenylpropyl)-2-(5-N-morpholino-3,3-dimethylpentyl)-thiophene and its hydrochloride,

5-(3-m-fluorophenylpropyl)-2-(5-N-morpholino-3,3-dimethylpentyl)-thiophene and its hydrochloride,
5-(3-o-chlorophenylpropyl)-2-(5-N-morpholino-3,3-dimethylpentyl)-thiophene and its hydrochloride,
5-(3-phenylpropyl)-2-(5-N-morpholino-3,3-tetramethylenepentyl)-thiophene and its hydrochloride,
5-(3-p-methylphenylpropyl)-2-(5-N-morpholino-4,4-dimethylpentyl)-thiophene and its hydrochloride.

**3.** The compounds of claim 1 which are:
5-(3-p-fluorophenylpropyl)-2-{5-[4-(2,3,4-trimethoxybenzyl)-piperazinyl]-3,3-dimethylpentyl}-thiophene and its dihydrochloride,
5-(3-phenylpropyl)-2-{5-[4-(2,3,4-trimethoxybenzyl)-piperazinyl]-3,3-dimethylpentyl}-thiophene and its dihydrochloride,
5-(3-m-fluorophenylpropyl)-2-{5-[4-(2,3,4-trimethoxy-benzyl)-piperazinyl]-3,3-dimethylpentyl}-thiophene and its dihydrochloride,
5-(3-p-methylphenylpropyl)-2-{5-[4-(2,3,4-trimethoxybenzyl)-piperazinyl]-3,3-dimethylpentyl}-thiophene and its dihydrochloride,
5-(3-m-fluorophenylpropyl)-2-{5-[4-(2,3,4-trimethoxybenzyl)-piperazinyl]-3-methylpentyl}-thiophene,
5-(2-p-methylphenylethyl)-2-{5-[4-(2,3,4-trimethoxybenzyl)-piperazinyl]-3-methylpentyl}-thiophene,
5-(2-p-methylphenylethyl)-2-{5-[4-(2,3,4-trimethoxybenzyl)-piperazinyl]-3,3-dimethylpentyl}-thiophene,
5-(6-phenylhexyl)-2-{5-[4-(2,3,4-trimethoxybenzyl)-piperazinyl]-3,3-dimethylpentyl}-thiophene,
5-[3-(2,4-dimethylphenyl-)propyl]-2-{5-[4-(2,3,4-trimethoxybenzyl)-piperazinyl]-3-methylpentyl}-thiophene,
5-[3-(2,4-dimethylphenyl)-propyl]-2-{5-[4-(2,3,4-trimethoxybenzyl)-piperazinyl]-3,3-dimethylpentyl}-thiophene,
5-[3-(3,5-dimethylphenyl)-propyl]-2-{5-[4-(2,3,4-trimethoxybenzyl)-piperazinyl]-3-methylpentyl)-thiophene,
5-[3-(3,5-dimethylphenyl)-propyl]-2-{5-[4-(2,3,4-trimethoxybenzyl)-piperazinyl]-3,3-dimethylpentyl}-thiophene,
5-(4-p-methylphenylbutyl)-2-{5-[4-(2,3,4-trimethoxybenzyl)-piperazinyl]-3-methylpentyl}-thiophene,
5-(4-p-methylphenylbutyl)-2-{5-[4-(2,3,4-trimethoxybenzyl)-piperazinyl]-3,3-dimethylpentyl}-thiophene.

**4.** A process for the preparation of the compounds of claim 1, characterised in that :
an acid of the general formula II :

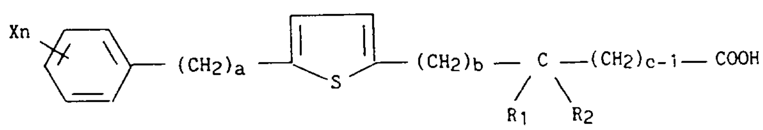

(II)

in which X, n, a, b, c, $R_1$ and $R_2$ are as defined in claim 1,
is converted into the corresponding acid chloride of the general formula III :

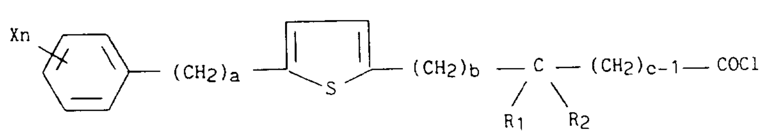

(III)

in which X n, a, b, c, $R_1$ and $R_2$ are as defined in claim 1,
which acid chloride is used to acylate an amine of the general formula IV :

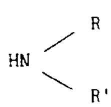

(IV)

in which R and R' are as defined in claim 1,

and the amide so-obtained of the general formula V :

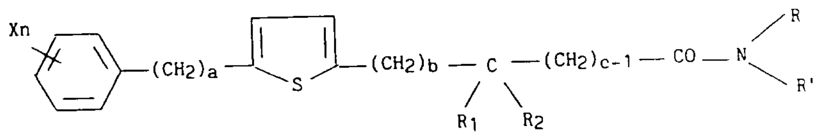

(V)

in which X $\underline{n}$, $\underline{a}$, $\underline{b}$, $\underline{c}$, $R_1$, $R_2$, R and R' are as defined in claim 1 is reduced.

**5.** Pharmaceutical compositions containing as active ingredient a compound according to claims 1 to 3 together with appropriate pharmaceutical excipients.

**6.** Pharmaceutical compositions according to claim 5, in a form suitable in particular for the treatement of pathologies that are characterised by a loss of bone tissue.

**7.** The amides of the general formula V :

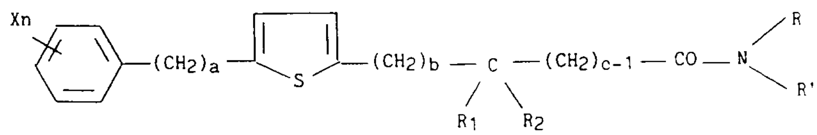

(V)

wherein X, $\underline{n}$, $\underline{a}$, $\underline{b}$, $\underline{c}$, $R_1$, $R_2$, R and R' are as defined in claim 1 as new industrial products that can be used in the synthesis of the compounds of formula I according to claim 1.

**Claims for the following Contracting States : ES, GR**

**1.** A process for the preparation of thiophene compounds of the general formula I :

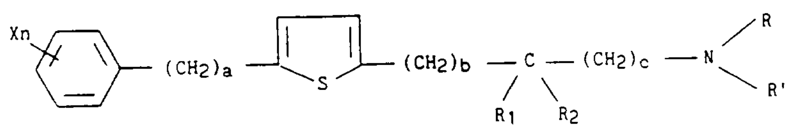

(I)

in which :

- X represents a hydrogen or halogen atom, a straight-chain or branched alkyl or alkoxy radical each having from 1 to 5 carbon atoms, or a dialkylamino radical in which each alkyl group contains from 1 to 5 carbon atoms;
- $\underline{n}$ represents 1 or 2;
- $\underline{a}$ represents an integer of from 2 to 6 inclusive;
- $\underline{b}$ represents 2 or 3;
- $\underline{c}$ represents 1 or 2, and is such that $\underline{b}+\underline{c} = 4$;
- $R_1$ and $R_2$, which may be the same or different, each represents a hydrogen atom or an alkyl radical having from 1 to 5 carbon atoms, or
  $R_1$ and $R_2$ together with the carbon atom to which they are bonded form a hydrocarbon ring containing from 3 to 6 carbon atoms; and
- R and R', which may be the same or different, each represents a hydrogen atom or an alkyl radical having from 1 to 5 carbon atoms, or
  R and R' together with the nitrogen atom to which they are bonded form a pentagonal or hexagonal heterocyclic radical optionally containing an oxygen atom or a second nitrogen atom which may itself be substituted by an alkyl radical containing from 1 to 5 carbon atoms or by an arylalkyl radical in which the alkyl group contains from 1 to 5 carbon atoms and the aryl group is either unsubstituted or mono- or poly-substituted by a halogen atom or an alkyl or alkoxy radical each having from 1 to 5 carbon atoms,

and the physiologically tolerable addition salts thereof with appropriate acids,

characterised in that :

- an acid of the general formula II :

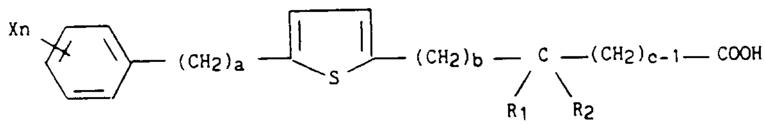

(II)

in which X n, a, b, c $R_1$ and $R_2$ are as defined above,
is converted into the corresponding acid chloride of the general formula III :

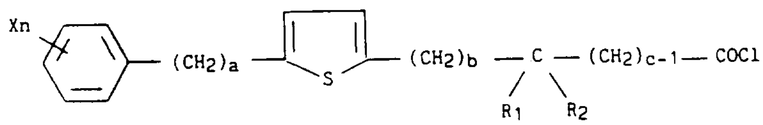

(III)

in which X, n, a, b, c, $R_1$ and $R_2$ are as defined above, which acid chloride is used to acylate an amine of the general formula IV :

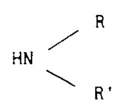

(IV)

in which R and R' are as defined above,
and the amide so-obtained of the general formula V :

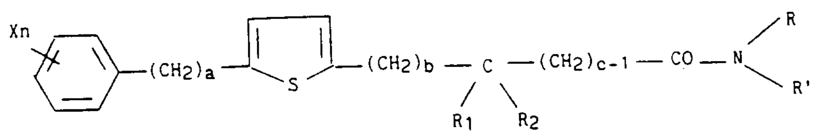

(V)

in which X n, a, b, c, $R_1$, $R_2$, R and R' are as defined above, is reduced;
and, if desired, the compounds so obtained are treated with appropriate acids to yield the corresponding addition salts.

**2.** A process for the preparation of intermediates of the synthesis of the compounds of the general formula I, which intermediates correspond to the general formula V :

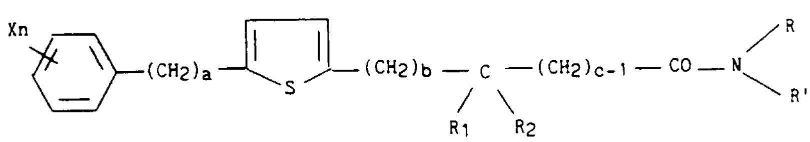

(V)

wherein X, n, a, b, c, $R_1$, $R_2$, R and R' are as defined in claim 1,

- characterised in that the procedure is as included in claim 1, starting from the compound II up to obtaining the amides V.

**Patentansprüche**

**Patentansprüche für folgende Vertragsstaaten : AT, BE, CH, DE, DK, FR, GB, IT, LI, LU, NL, SE**

1. Thiophenderivate der allgemeinen Formel I:

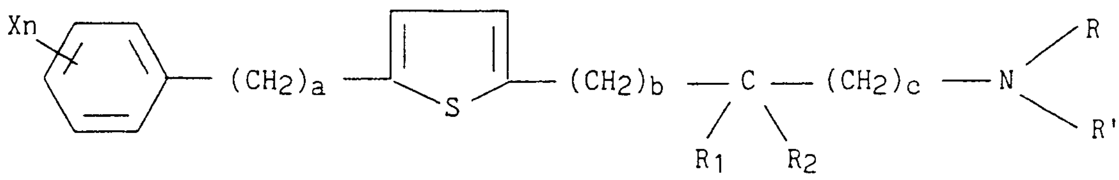

(I)

in der:

- X ein Wasserstoff- oder Halogenatom, eine Alkyl- oder Alkoxygruppe mit jeweils 1 bis 5 Kohlenstoffatomen in gerader oder verzweigter Kette oder eine Dialkylaminogruppe, worin jede Alkylgruppe 1 bis 5 Kohlenstoffatome umfaßt;
- n 1 oder 2;
- a eine ganze Zahl mit einem Wert von 2 bis 6 einschließlich;
- b 2 oder 3;
- c 1 oder 2, mit der Maßgabe, daß b + c = 4;
- $R_1$ und $R_2$, die gleichartig oder verschieden sein können, jeweils ein Wasserstoffatom oder eine Alkylgruppe mit 1 bis 5 Kohlenstoffatomen oder $R_1$ und $R_2$ gemeinsam mit dem Kohlenstoffatom, an das sie gebunden sind, einen Kohlenwasserstoffring mit 3 bis 6 Kohlenstoffatomen; und
- Rund R', die gleichartig oder verschieden sein können, jeweils ein Wasserstoffatom oder eine Alkylgruppe mit 1 bis 5 Kohlenstoffatomen oder R und R' gemeinsam mit dem Stickstoffatom, an das sie gebunden sind, einen penta- oder hexagonalen heterocyclischen Rest, der gegebenenfalls ein Sauerstoffatom oder ein zweites Stickstoffatom enthält und der seinerseits durch eine Alkylgruppe mit 1 bis 5 Kohlenstoffatomen oder eine Arylalkylgruppe, worin die Alkylgruppe 1 bis 5 Kohlenstoffatome aufweist und die Arylgruppe entweder nichtsubstituiert oder durch ein Halogenatom oder eine Alkyl- oder Alkoxygruppe mit jeweils 1 bis 5 Kohlenstoffatomen mono- oder polysubstituiert sein kann, substituiert sein kann,

bedeuten
und deren physiologisch verträgliche Additionssalze mit geeigneten Säuren.

2. Verbindungen nach Anspruch 1, nämlich:
5-(2-Phenyl-ethyl)-2-(5-N-morpholino-3,3-dimethyl-pentyl)-thiophen und dessen Hydrochlorid,
5-(2-p-Methylphenyl-ethyl)-2-(5-N-morpholino-3,3-dimethyl-pentyl)-thiophen und dessen Hydrochlorid,
5-(3-Phenyl-propyl)-2-(5-N-morpholino-pentyl)-thiophen und dessen Methansulfonat,
5-(3-p-Methylphenyl-propyl)-2-(5-N-morpholino-pentyl)-thiophen und dessen Hydrochlorid,
5-(3-Phenyl-propyl)-2-(5-N-morpholino-3-methyl-pentyl)-thiophen und dessen Hydrochlorid,
5-(3-p-Methylphenyl-propyl)-2-(5-N-morpholino-3-methyl-pentyl)-thiophen und dessen Hydrochlorid,
5-(3-p-Fluorphenyl-propyl)-2-(5-N-morpholino-3-methyl-pentyl)-thiophen und dessen Hydrochlorid,
5-(3-Phenyl-propyl)-2-(5-N-morpholino-3,3-dimethyl-pentyl)-thiophen und dessen Methansulfonat,
5-(3-p-Methylphenyl-propyl)-2-(5-N-morpholino-3,3-dimethyl-pentyl)-thiophen und dessen Hydrochlorid,
5-(3-p-Fluorphenyl-propyl)-2-(5-N-morpholino-3,3-dimethyl-pentyl)-thiophen und dessen Hydrochlorid,
5-(3-m-Fluorphenyl-propyl)-2-(5-N-morpholino-3,3-dimethyl-pentyl)-thiophen und dessen Hydrochlorid,
5-(3-o-Chlorphenyl-propyl)-2-(5-N-morpholino-3,3-dimethyl-pentyl)-thiophen und dessen Hydrochlorid,
5-(3-Phenyl-propyl)-2-(5-N-morpholino-3,3-tetramethylen-pentyl)-thiophen und dessen Hydrochlorid und
5-(3-p-Methylphenyl-propyl)-2-(5-N-morpholino-4,4-dimethyl-pentyl)-thiophen und dessen Hydrochlorid.

3. Die Verbindungen nach Anspruch 1, nämlich:
5-(3-p-Fluorphenyl-propyl)-2-{5-[4-(2,3,4-trimethoxy-benzyl)-piperazinyl]-3,3-dimethyl-pentyl}-thiophen und dessen Dihydrochlorid,
5-(3-Phenyl-propyl)-2-{5-[4-(2,3,4-trimethoxy-benzyl)-piperazinyl]-3,3-dimethyl-pentyl}-thiophen und dessen Dihydrochlorid,
5-(3-m-Fluorphenyl-propyl)-2-{5-14-(2,3,4-trimethoxy-benzyl)-piperazinyl]-3,3-dimethyl-pentyl}-thiophen und dessen Dihydrochlorid,

5-(3-p-Methylphenyl-propyl)-2-{5-[4-(2,3,4-trimethoxy-benzyl)-piperazinyl]-3,3-dimethyl-pentyl}-thiophen und dessen Dihydrochlorid,
5-[3-m-Fluorphenyl-propyl)-2-{5-[4-(2,3,4-trimethoxy-benzyl)-piperazinyl]-3-methyl-pentyl}-thiophen,
5-(2-p-Methylphenyl-ethyl)-2-{5-[4-(2,3,4-trimethoxy-benzyl)-piperazinyl]-3-methyl-pentyl}-thiophen,
5-(2-p-Methylphenyl-ethyl)-2-{5-[4-(2,3,4-trimethoxy-benzyl)-piperazinyl]-3,3-dimethyl-pentyl}-thiophen,
5-(6-Phenyl-hexyl)-2-{5-[4-(2,3,4-trimethoxy-benzyl)-piperazinyl]-3,3-dimethyl-pentyl}-thiophen,
5-[3(2,4-Dimethyl-phenyl)-propyl]-2-{5-[4-(2,3,4-trimethoxy-benzyl)-piperazinyl]-3-methyl-pentyl}-thiophen,
5-[3-(2,4-Dimethyl-phenyl)-propyl]-2-{5-14-(2,3,4-trimethoxy-benzyl)-piperazinyl]-3,3-dimethyl-pentyl}-thiophen,
5-[3-(3,5-Dimethyl-phenyl)-propyl]-2-{5-[4-(2,3,4-trimethoxy-benzyl)-piperazinyl]-3-methyl-pentyl}-thiophen,
5-[3-(3,5-Dimethyl-phenyl)-propyl]-2-{5-[4-(2,3,4-trimethoxy-benzyl)-piperazinyl]-3,3-dimethyl-pentyl}-thiophen,
5-(4-p-Methylphenyl-butyl)-2-{5-[4-(2,3,4-trimethoxy-benzyl)-piperazinyl]-3-methyl-pentyl}-thiophen und
5-(4-p-Methylphenyl-butyl)-2-{5-[4-(2,3,4-trimethoxy-benzyl)-piperazinyl]-3,3-dimethyl-pentyl}-thiophen.

4. Verfahren zur Herstellung der Derivate nach Anspruch 1, **dadurch gekennzeichnet,** daß man die Säure der allgemeinen Formel II:

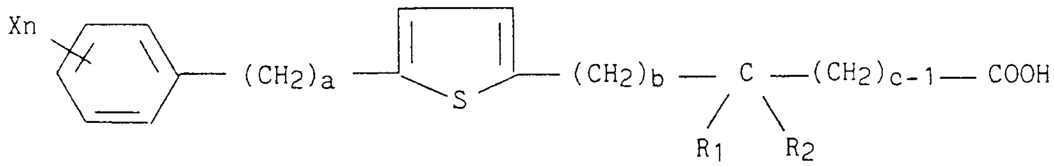

(II)

in der X, n, a, b, c, $R_1$ und $R_2$ die in Anspruch 1 angegebenen Bedeutungen besitzen, in das entsprechende Säurechlorid der allgemeinen Formel III:

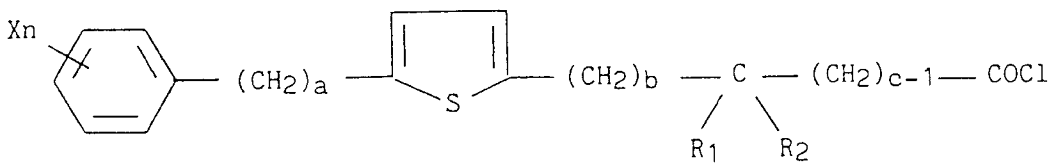

(III)

umwandelt, in der X, n, a, b, c, $R_1$ und $R_2$ die in Anspruch 1 angegebenen Bedeutungen besitzen, welches Säurechlorid dazu dient, das Amin der allgemeinen Formel IV:

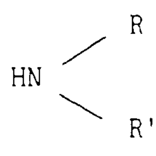

(IV)

in der R und R' die in Anspruch 1 angegebenen Bedeutungen besitzen, zu acylieren, und man das in dieser Weise erhaltene Amid der allgemeinen Formel V:

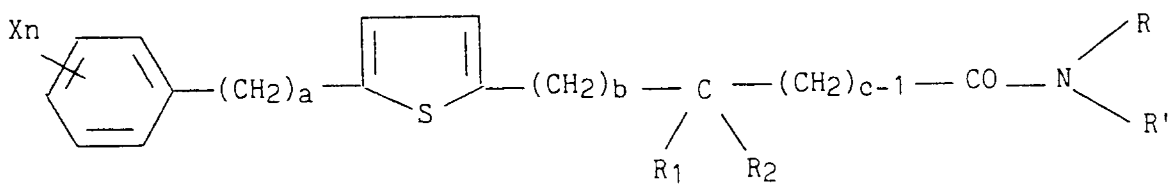

(V)

in der X, n, a, b, c, $R_1$, $R_2$, R und R' die in Anspruch 1 angegebenen Bedeutungen besitzen, reduziert.

**5.** Pharmazeutische Zubereitungen enthaltend als Wirkstoff ein Derivat nach den Ansprüchen 1 bis 3 zusammen mit geeigneten pharmazeutischen Trägermaterialien.

**6.** Pharmazeutische Zubereitungen nach Anspruch 5 in einer Form, die insbesondere zur Behandlung von pathologischen Zuständen geeignet ist, die durch einen Verlust des Knochengewebes gekennzeichnet sind.

**7.** Als neue technische Produkte, die bei der Synthese der Derivate der Formel I nach Anspruch 1 geeignet sind, nämlich die Amide der Formel V:

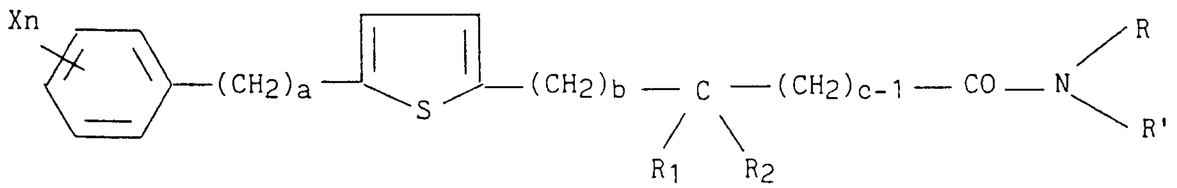

(V)

in der X, n, a, b, c, $R_1$, $R_2$, R und R' die in Anspruch 1 angegebenen Bedeutungen besitzen.

**Patentansprüche für folgende Vertragsstaaten : ES, GR**

**1.** Verfahren zur Herstellung von Thiophenderivaten der allgemeinen Formel I:

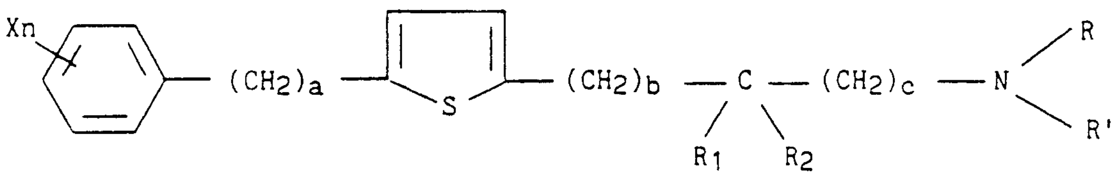

(I)

in der:

- X ein Wasserstoff- oder Halogenatom, eine Alkyl- oder Alkoxygruppe mit jeweils 1 bis 5 Kohlenstoffatomen in gerader oder verzweigter Kette oder eine Dialkylaminogruppe, worin jede Alkylgruppe 1 bis 5 Kohlenstoffatome umfaßt;
- n 1 oder 2;
- a eine ganze Zahl mit einem Wert von 2 bis 6 einschließlich;
- b 2 oder 3;
- c 1 oder 2, mit der Maßgabe, daß b + c = 4;
- $R_1$ und $R_2$, die gleichartig oder verschieden sein können, jeweils ein Wasserstoffatom oder eine Alkylgruppe mit 1 bis 5 Kohlenstoffatomen oder $R_1$ und $R_2$ gemeinsam mit dem Kohlenstoffatom, an das sie gebunden sind, einen Kohlenwasserstoffring mit 3 bis 6 Kohlenstoffatomen; und
- Rund R', die gleichartig oder verschieden sein können, jeweils ein Wasserstoffatom oder eine Alkylgruppe mit 1 bis 5 Kohlenstoffatomen oder Rund R' gemeinsam mit dem Stickstoffatom, an das sie gebunden sind, einen penta- oder hexagonalen heterocyclischen Rest, der gegebenenfalls ein Sauerstoffatom oder ein zweites Stickstoffatom enthält und der seinerseits durch eine Alkylgruppe mit 1 bis 5 Kohlenstoffatomen oder eine Arylalkylgruppe, worin die Alkylgruppe 1 bis 5 Kohlenstoffatome aufweist und die Arylgruppe entweder nichtsubstituiert oder durch ein Halogenatom oder eine Alkyl- oder Alkoxygruppe mit jeweils 1 bis 5 Kohlenstoffatomen mono- oder polysubstituiert sein kann, substituiert sein kann,

bedeuten

und von deren physiologisch verträglichen Salzen mit geeigneten Säuren, **dadurch gekennzeichnet,** daß man die Säure der allgemeinen Formel II:

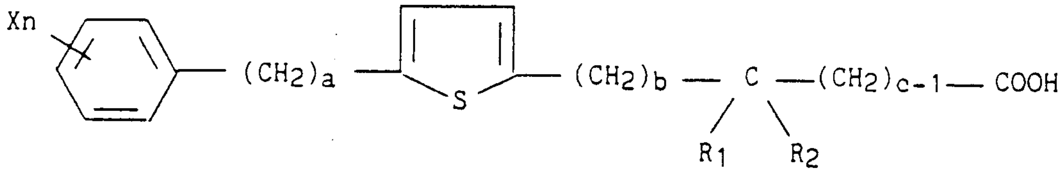

(II)

in der X, n, a, b, c, $R_1$ und $R_2$ die oben angegebenen Bedeutungen besitzen, in das entsprechende Säurechlorid der allgemeinen Formel III:

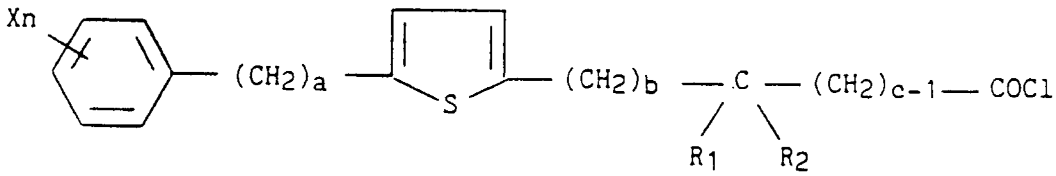

(III)

umwandelt, In der X, n, a, b, c, $R_1$ und $R_2$ die oben angegebenen Bedeutungen besitzen, welches Säurechlorid dazu dient, das Amin der allgemeinen Formel IV:

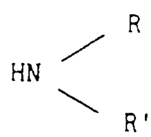

(IV)

in der R und R' die oben angegebenen Bedeutungen besitzen, zu acylieren, und man das in dieser Weise erhaltene Amid der allgemeinen Formel V:

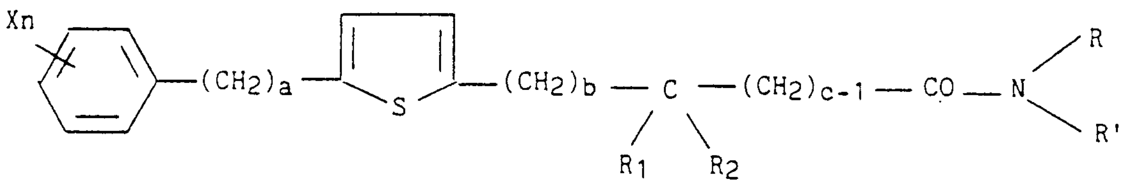

(V)

in der X, n, a, b, c, $R_1$, $R_2$, R und R' die oben angegebenen Bedeutungen besitzen, reduziert; und gewünschtenfalls die In dieser Weise erhaltenen Derivate mit geeigneten Säuren behandelt, um die entsprechenden Additionssalze zu bilden.

**2.** Verfahren zur Herstellung der Zwischenprodukte der Synthese der Derivate der allgemeinen Formel I, welche Zwischenprodukte der allgemeinen Formel V entsprechen:

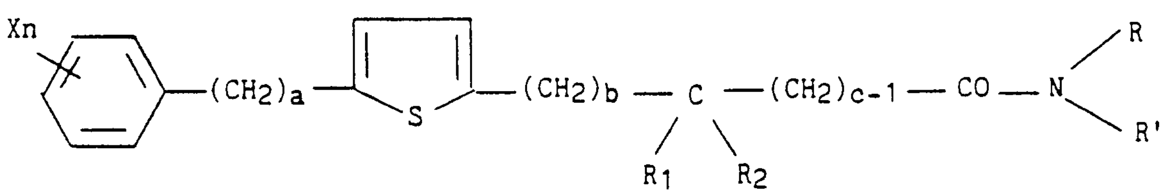

(V)

in der X, n, a, b. c, $R_1$, $R_2$, R und R' die in Anspruch 1 angegebenen Bedeutungen besitzen, **dadurch gekennzeichnet,** daß man nach der in Anspruch 1 angegebenen Verfahrensweise ausgehend von den Derivaten der Formel I erfährt, bis man die Amide V erhalten hat.